(19) Europäisches Patentamt
European Patent Office
Office européen des brevets

(11) **EP 0 556 330 B1**

(12) **EUROPEAN PATENT SPECIFICATION**

(45) Date of publication and mention
of the grant of the patent:
**22.05.2002 Bulletin 2002/21**

(51) Int Cl.[7]: **C12N 15/11**, C12P 19/34,
C12Q 1/68

(21) Application number: **92901270.6**

(22) Date of filing: **08.11.1991**

(86) International application number:
**PCT/US91/08200**

(87) International publication number:
**WO 92/08791 (29.05.1992 Gazette 1992/12)**

(54) **METHOD OF TARGETING DNA**

METHODEN ZUR ZIELRICHTUNG VON DNA

PROCEDE DE CIBLAGE D'ADN

(84) Designated Contracting States:
**AT BE CH DE DK ES FR GB GR IT LI LU NL SE**

(30) Priority: **09.11.1990 US 611268**
**24.07.1991 US 733744**

(43) Date of publication of application:
**25.08.1993 Bulletin 1993/34**

(73) Proprietor: **The Government of the United States**
**of America, as represented by the Secretary,**
**Department of Health and Human Services**
**Washington, D.C. 20201 (US)**

(72) Inventors:
 • **CAMERINI-OTERO, Rafael, D.**
 **Kensington, MD 20895 (US)**
 • **MCINTOSH, Margaret**
 **Chevy Chase, MD 20815 (US)**
 • **CAMERINI-OTERO, Carol, S.**
 **Kensington, MD 20895 (US)**
 • **FERRIN, Lance, J. 12411 Branfield Court**
 **Rockville, MD 20852 (US)**

(74) Representative: **Gillard, Marie-Louise et al**
**Cabinet Beau de Loménie**
**158, rue de l'Université**
**75340 Paris Cédex 07 (FR)**

(56) References cited:
**US-A- 4 888 274**

• **NIELSEN, P.E. 'Photochemical probes in**
**biochemistry'; 1989, KLUWER ACADEMIC**
**Publishers pages 169-177 CHENG, S. et al.**
**'RecA-directed hybridization of**
**psoralen-monoadducted DNA oligonucleotides**
**to duplex targets'**
• **JOURNAL OF THE NATIONAL CANCER**
**INSTITUTE, vol.81, no.20, 18 October 1989,**
**BETHESDA, MD, US pages 39 - 44**
**ROTHENBERG, M. ET AL.**
**'Oligodeoxynucleotides as anti-sense inhibitors**
**of gene expression: therapeutic implications'**
• **PROCEEDINGS OF THE NATIONAL ACADEMY**
**OF SCIENCES OF USA, vol.85, no.4, February**
**1988, WASHINGTON US pages 1349 - 1353**
**PRASEUTH, D. ET AL. 'Sequence-specific**
**binding and photocrosslinking of alpha and beta**
**oligodeoxynucleotides to the major groove of**
**DNA via triple-helix formation'**
• **GENES DEV 4 (11). 13 NOVEMBER 1990.**
**1951-1963 HSIEH, P. ET AL. 'PAIRING OF**
**HOMOLOGOUS DNA SEQUENCES BY**
**PROTEINS EVIDENCE FOR THREE-STRANDED**
**DNA.'**
• **Journal of Molecular Biology, Volume 193,**
**issued 1987, S.C. KOWALCZYKOWSKI et al,**
**"Effects of Escherichia Coli SSB Protein on the**
**Single-Stranded DNA-Dependent Atpase**
**Activity of Escherichia col: Reca Protein", pages**
**97-113, see entire document.**
• **L. STRYER "Biochemistry", Second Edition,**
**published 1981 by W.H. Freeman and Company**
**(San Francisco) pages 754-756, see entire**
**document.**

- The Journal of Biological Chemistry , Volume 264, No. 9. issued 25 March 1989, HSIEH et al, "Formation of Joint DNA Molecules by Two Eukaryotic Strand Exchange Proteins does not Require Melting of a DNA Duplex," pages 5089-5097, see entire document.
- Science, Volume 245, issued 18 August 1989, L.J. MAHER III et al, "Inhibition of DNA Binding Proteins by Oligonucleotide-Directed Triple Helix Formation", pages 725-730, see entire document.
- Biochemistry, Volume 28, issued 1989, J-C FRANCOIS et al., "Inhibition of Restriction Endonuclease Cleavage via Triple Helix Formation by Homopyrimidine Oligonucleotides", pages 9617-9619, see entire document.

- Science, Volume 241, issued 22 July 1988, M. COONEY et al, "Site-Specific Oligonucleotide binding Represses Transcription of the Human C-MYC gene in Vitro", pages 456-459, see entire document.
- Proceedings of the National Academy of Sciences, U.S.A. Volume 82, issued February 1985, G.B. DREYER et al, "Sequence-Specific Cleavage of Single-Stranded DNA: Oligodeoxynucleotide-EDTA-Fe (II)", pages 968-972, see entire document.
- The Journal of Biological Chemistry, Volume 265, No. 28, issued 05 October 1990, UMLAUF et al, "Triple-Helical DNA Pairing Intermediates formed by RecA Protein", pages 16898-16912, see entire document.

## Description

This is a continuation-in-part of Application No. 07/611,268, filed November 9, 1990, the entire contents of which are incorporated herein by reference.

BACKGROUND ON THE INVENTION

Technical Field

The present invention relates, in general, to a method of forming triplex DNA and to a method of achieving sequence specific cleavage of duplex DNA using such a triplex.

Background Information

Several groups have recently reported highly efficient cleavage of genomic DNA at specific sequences. For example, Szybalski and coworkers have cleaved Saccharomyces cerevisiae and E. coli genomes at a single introduced lac operator site (Koob et al, *Science* 250, 271 (1990)). These investigators first methylated Hae II sites in the DNA while using the lac repressor to protect the lac operator from methylation. After inactivation of the methylase and the repressor, the only Hae II site unmodified and available for cleavage was the lac operator site. The advantages of this approach were the high yield and high specificity. The disadvantage was that only a lac operator site could be cleaved.

A second approach was used by several investigators to cleave genomes as large as S. cerevisiae. This approach used the ability of synthetic homopyrimidine oligonucleotides to anneal to duplex homopyrimidine-homopurine tracts to form triple-helical structures. This approach was first used by Moser and Dervan *(Science* 238, 645 (1987)) to cleave a plasmid by equipping the oligonucleotide with an EDTA-Fe cleavage moiety. Subsequently, other cleavage moieties were attached to homopyrimidine oligonucleotides. For example, Schultz and coworkers attached staphylococcal nuclease (Pei et al, *Proc. Natl. Acad. Sci. USA* 87, 9858 (1990)), and Helene and coworkers attached a phenanthroline-copper derivative (Francois et al, *Proc. Natl. Acad. Sci. USA* 86, 9702 (1989)) as cleavage moieties. Dervan and coworkers have also used a guanine-rich cleavage oligonucleotide to form a triplex (Beal et al, *Science* 251, 1360 (1991)), and have cleaved DNA using a triplex and the methylation protection strategy described above (Strobel et al, *Nature* 350, 172 (1991)). The advantages of this targeting approach are efficiency and the ability to use oligonucleotides with a variety of derivatives. The disadvantage is that only homopyrimidine or guanine-rich oligonucleotides have been used successfully.

The practical use of previously reported strategies is severely limited because of the paucity, or indeed the complete absence, of possible cleavage sites in any particular DNA sequence. The present invention, on the other hand, provides a general method of cleaving DNA at any desired site, or pair of sites. Site-specific cleavage, however, is only a single embodiment of the present invention. In a broader sense, the invention relates to a method of targeting any desired sequence specifically and efficiently whether, it be for purposes of cleavage, protection or enrichment.

SUMMARY OF THE INVENTION

It is a general object of the invention to provide method of cleaving DNA at any desired site, or pair of sites.

The method of the present invention comprises forming a three-stranded DNA molecule wherein each strand of the three-stranded DNA molecule is hybridized (that is, non-covalently bound) to at least one other strand of the three-stranded DNA molecule. The method comprises:

contacting a recombination protein with a double-stranded DNA molecule and with a single-stranded DNA molecule sufficiently complementary to one strand of the double-stranded DNA molecule to hybridize therewith, which contacting is effected under conditions such that the single-stranded DNA molecule hybridizes to the double-stranded molecule so that the three stranded DNA molecule is formed.

Further objects, and advantages, will become clear from a reading of the disclosure that follows.

BRIEF DESCRIPTION OF THE DRAWINGS

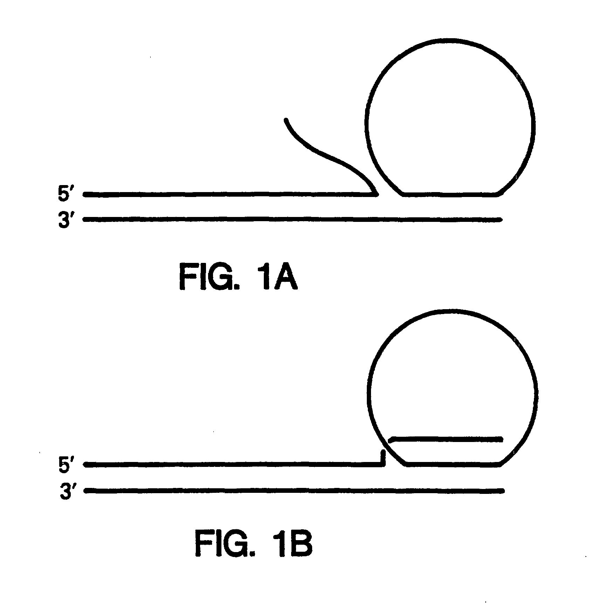

Figure 1 shows two possible joint molecule structures formed by recombinase protein between a linear duplex and a homologous single-strand circular DNA. Top, a joint molecule having heteroduplex DNA and a displaced strand. The presence of a displaced strand allows branch migration to occur resulting in rapid dissociation of the joint substrates when heteroduplex regions are short. Bottom, a stable joint molecule in which the three strands are associated by additional noncovalent interactions and cannot participate in branch migration. The arrangement of the three strands is only schematic, and no disruption of the starting duplex is implied.

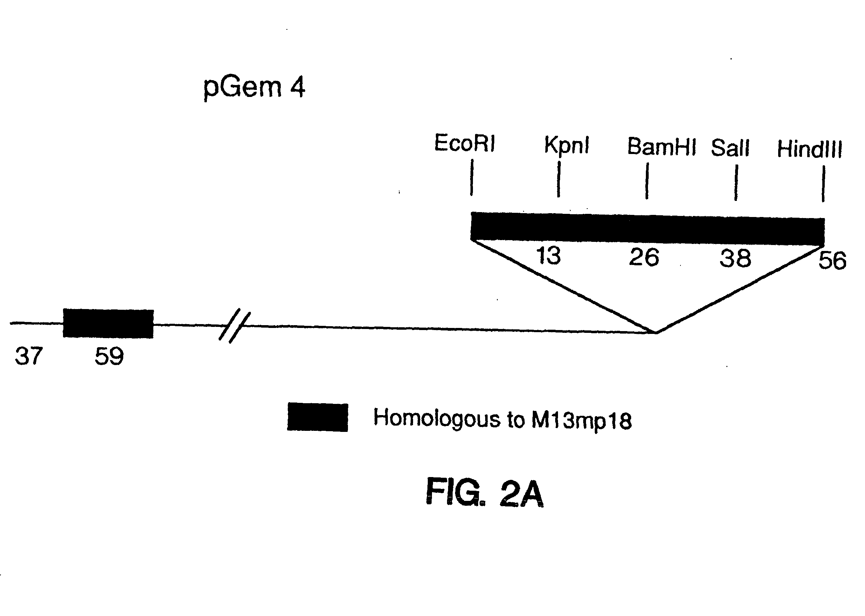

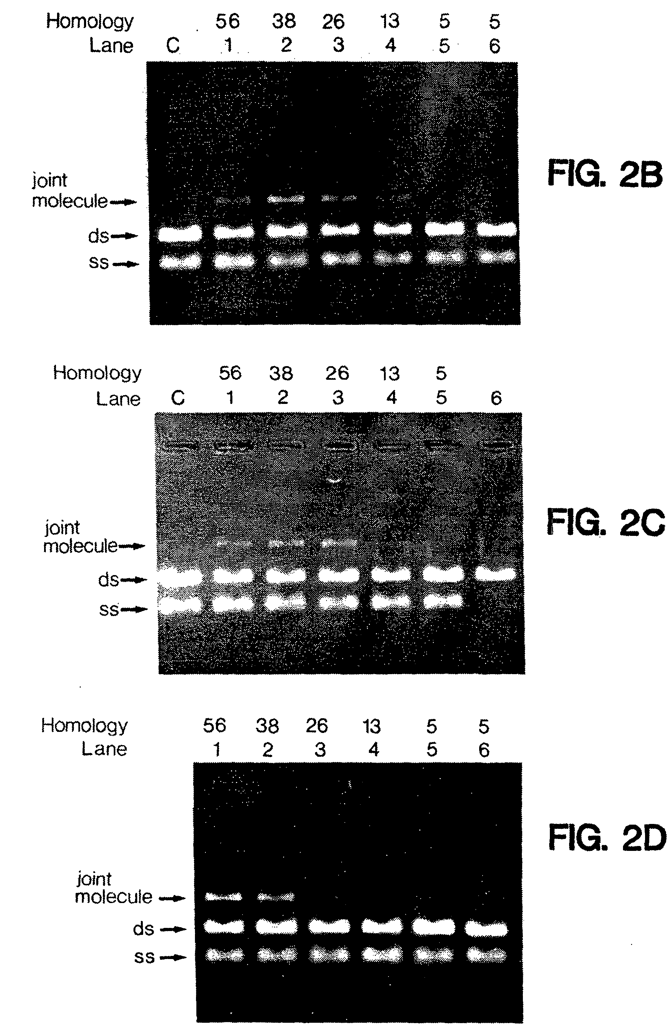

Figure 2 shows that recombinases form joint molecules using short homologies. A. partial map of the pGem 4 linear duplex substrate used in joint molecule assays is shown. Boxes indicate regions homologous to M13mp18. Numbers in the polylinker region at the right of the duplex indicate the lengths of homology available for pairing with M13mp18 single-strand DNA when pGem 4 is linearized at each of these sites. B - D. Joint molecule assays using pGem 4 linear duplex and M13mp18 viral strand DNA and HeLa recombinase fraction (panel B), Drosophila recombinase fraction (panel C) or E. coli recA and SSB proteins (panel D) were carried out as described in the Examples and were deproteinized by the addition of SDS. Lengths of homology available for pairing are indicated. Lane C, control, DNAs incubated alone; lane 1, Hind III-linearized pGem 4; lane 2, Sal I-linearized duplex; lane 3, Bam HI-linearized duplex; lane 4, Kpn I-linearized duplex; lane 5, Eco RI- linearized duplex; lane 6, Duplexes digested with both Hind III and Eco RI (panels B and D), or ligation control, duplex alone (panel C).

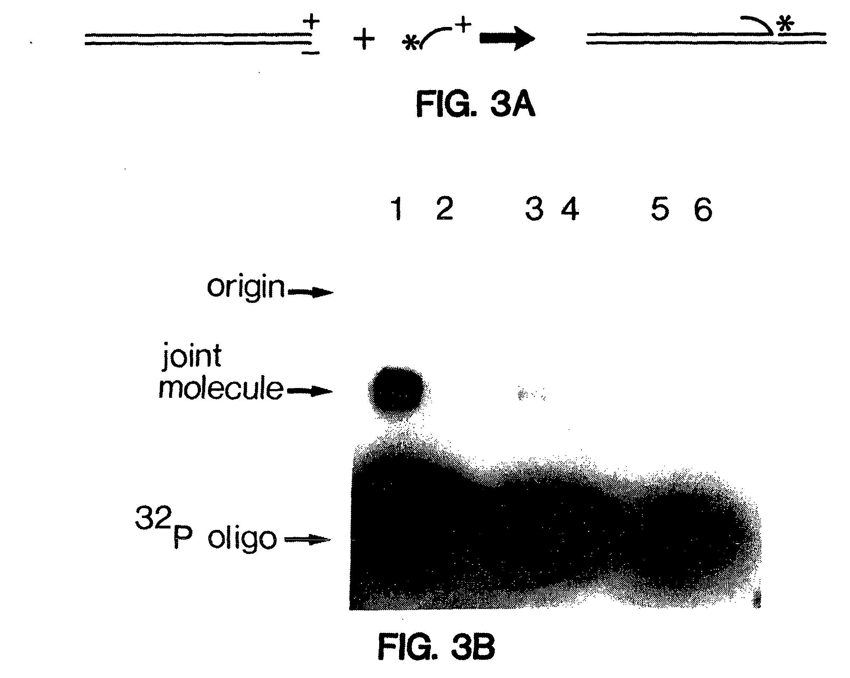

Figure 3 demonstrates novel joint molecule assay. A. Scheme for joint molecule formation between a linear duplex DNA and a homologous $^{32}$P-labeled oligonucleotide. B. Joint molecule assays were performed at 37°C for 10 min with HeLa recombinase fraction, 100 ng of Hind III-linearized pdel 9 duplex and 5 ng of a homologous $^{32}$P-labeled oligonucleotide 33 bases long (lane 1), 20 bases long (lane 3) or 13 bases long (lane 5). Samples were deproteinized with 1% SDS. Control experiments in lanes 2, 4 and 6 demonstrate that joint molecules are not formed by potential contaminating exonucleases. Duplexes and oligonucleotides (33-mer, lane 2, 20-mer, lane 4 and 13-mer, lane 6) were separately incubated with recombinase at 37°C, brought to 1% SDS and then combined and annealed at 65°C without quenching on ice prior to electrophoresis at room temperature. Under these annealing conditions, DNA duplexes 33 or 20 bp long can be formed and are stable.

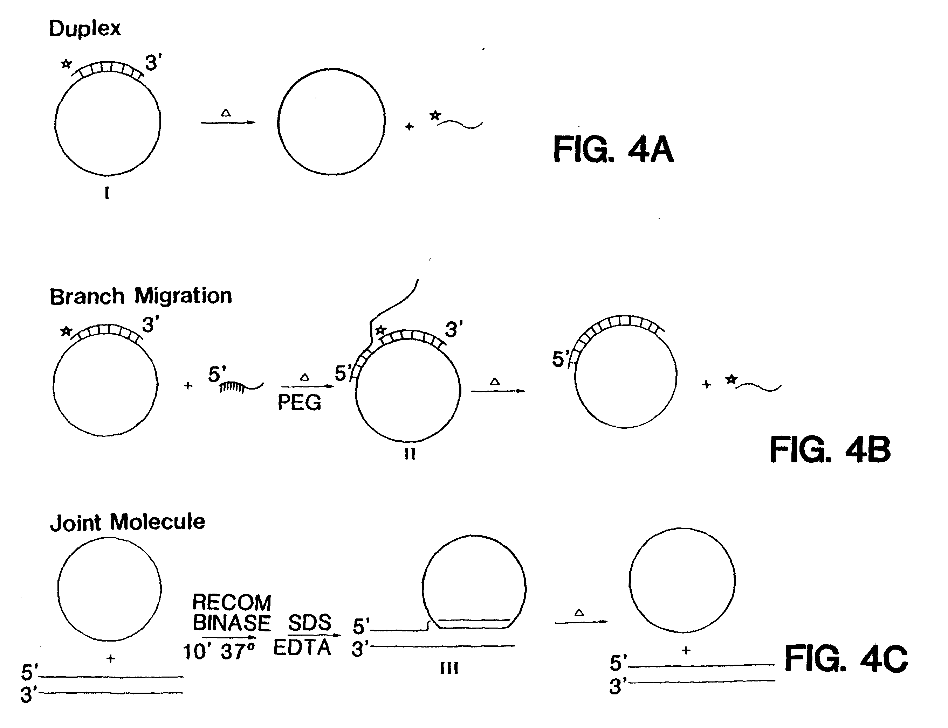

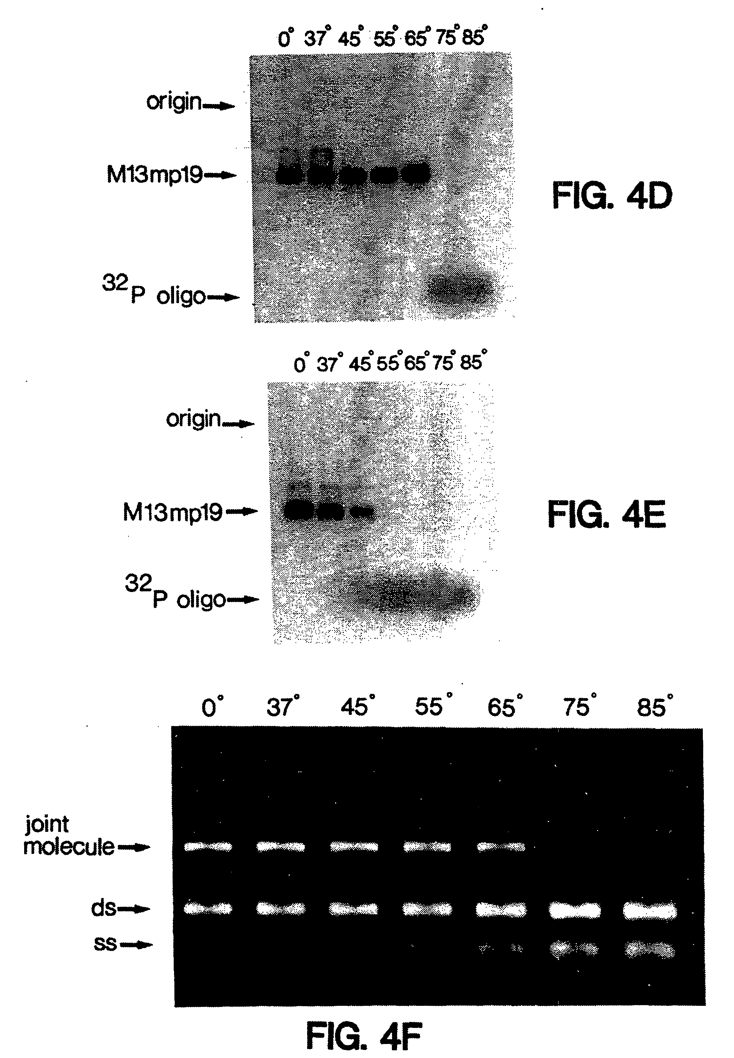

Figure 4 depicts the thermal stability of joint molecules. A-C. Scheme for determining relative thermal stabilities of short duplex regions, I, branch migration structures, II, or deproteinized joint molecules formed by recombinase, III. D-F. Representative data from thermal stability assays for short duplexes, branch migration structures and joint molecules involving a region of shared homology 38 base pairs long. Joint molecules were formed by HeLa recombinase fraction between M13mp18 single-strand DNA and Sal I-linearized pGem 4 duplex DNA and deproteinized by the addition of SDS. Stability of both the duplex and the branch migration structure was monitored after agarose gel electrophoresis as loss of $^{32}$P-label migrating at the position of M13mp19 single-strand DNA.

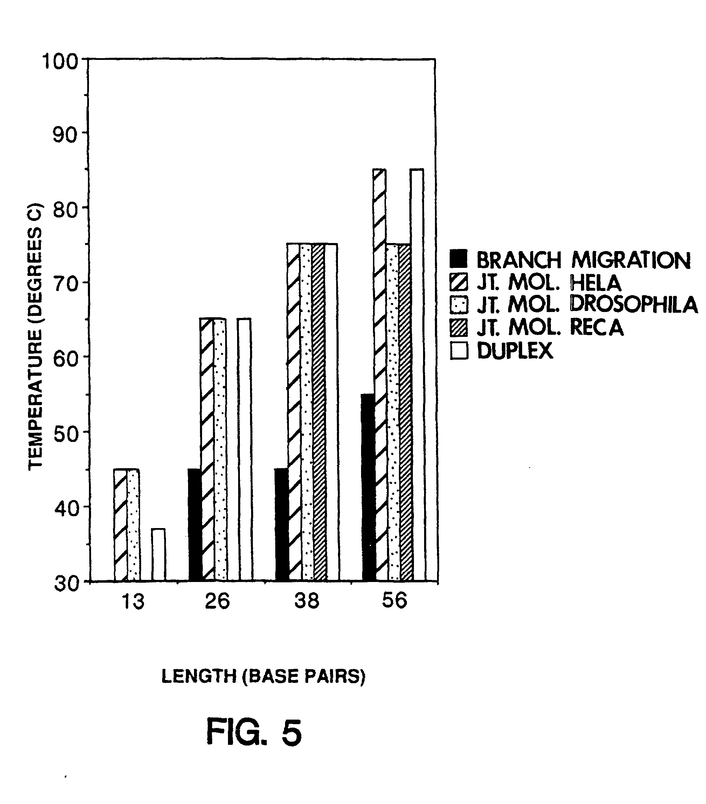

Figure 5 summarizes the thermal stability data. Thermal stability assays were carried out as described in the Examples for short duplexes, branch migration structures and joint molecules having 13, 26, 38 or 56 bp of homology. The indicated temperatures are those which resulted in dissociation of greater than 50% of the structures after a 10 min incubation. Because of the instability of the 13 bp duplex at 37°C, the stability of the corresponding 13 bp branch migration structure was not determined.

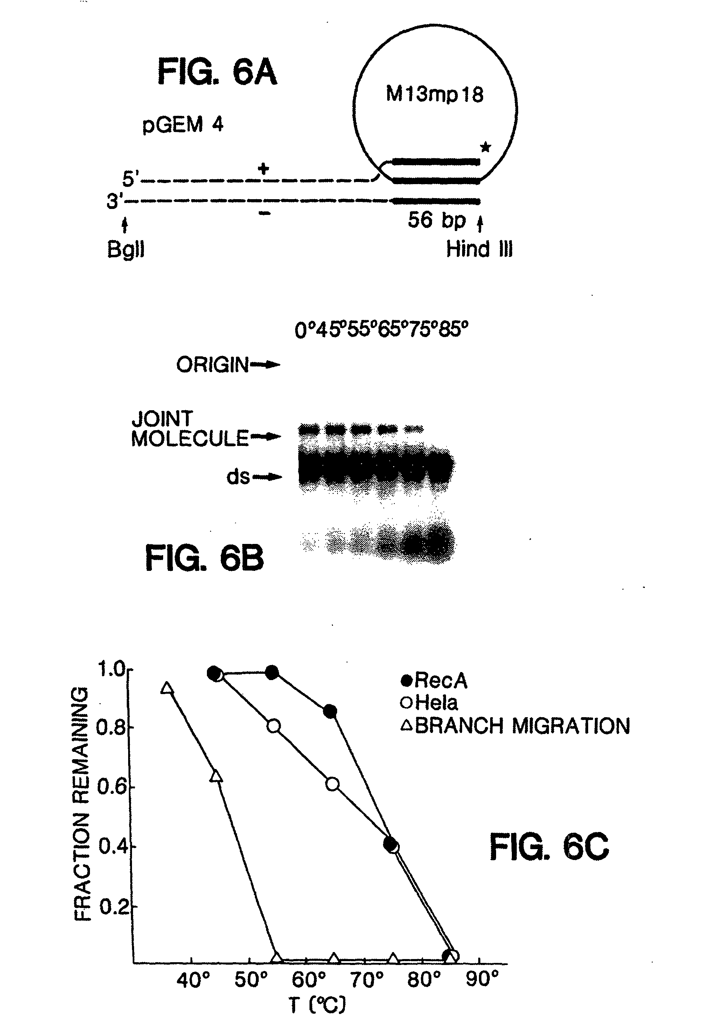

Figure 6 shows stable joint molecules having three intact DNA strands. A. A joint molecule formed from a Hind III-Bgl I fragment of pGem 4 having a unique $^{32}$P-label at the 3' end of the plus strand and sharing 56 bp of homology with M13mp18. B. Thermal stability assay of $^{32}$P-labeled joint molecules formed by recA protein. C. Comparison of the relative thermal stabilities of $^{32}$P-labeled joint molecules formed by E. coli recAprotein, filled circles, HeLa recombinase fraction, open circles, and the corresponding 56 bp branch migration structure, triangles.

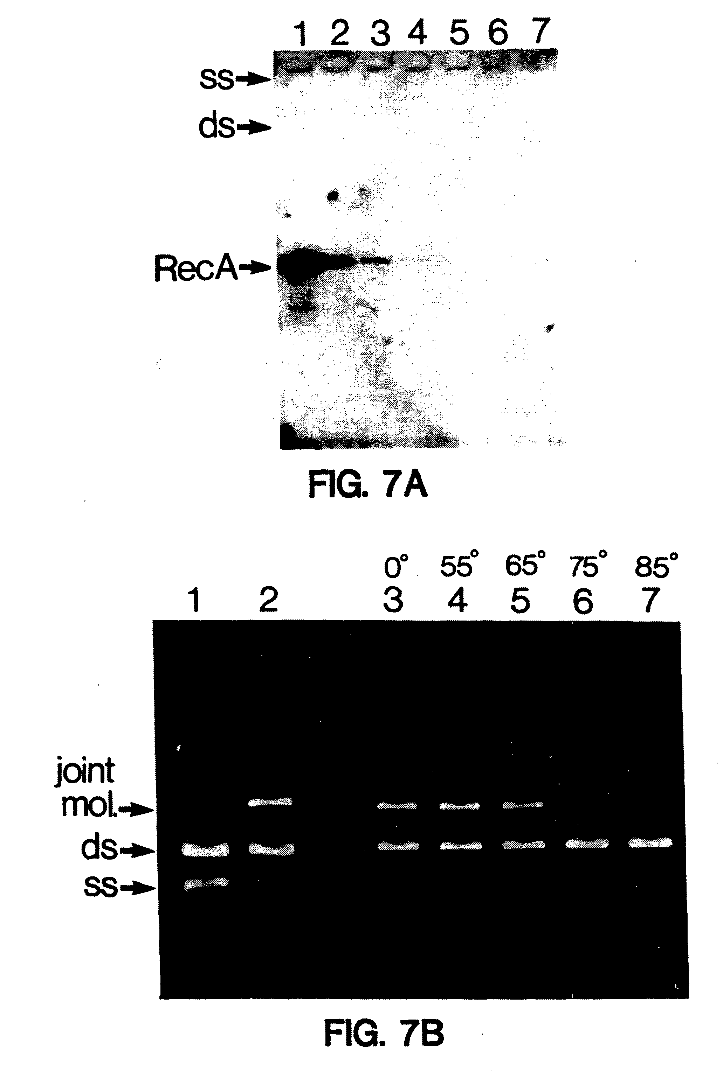

Figure 7 depicts stable joint molecules in the absence of recA protein. A. Joint molecules formed by recA were deproteinized as described in the Examples and analyzed for residual recA protein on a 12% SDS polyacrylamide gel followed by Western blotting with anti-recA antibody and $^{125}$I-labeled secondary antibody. Lanes 1-5, 5 ng, 1 ng, 0.5 ng, 0.2 ng, and 0.1 ng, respectively, of purified E.coli recA protein; lane 6, total deproteinized joint molecules from five assays; lane 7, 0.2 ng purified recA protein. B. Thermal stability assays of the deproteinized joint molecules. Lane 1, DNA substrates alone; lane 2, a reacted joint molecule assay with recA stopped with SDS and EDTA and loaded on the agarose gel directly; lanes 3-7, thermal stability assays of deproteinized joint molecules carried out as described in Figure 4. ds and ss indicate mobilities of double-strand and single-strand DNA, respectively.

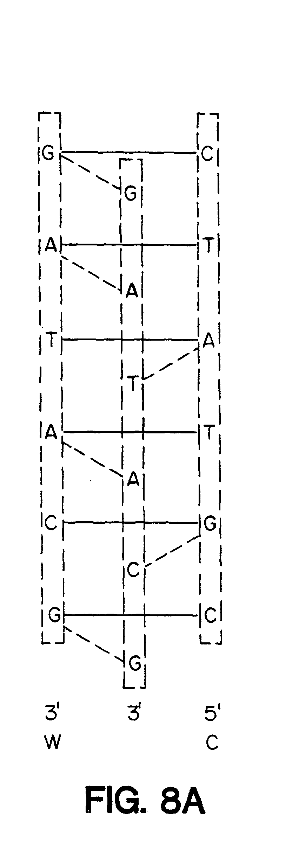

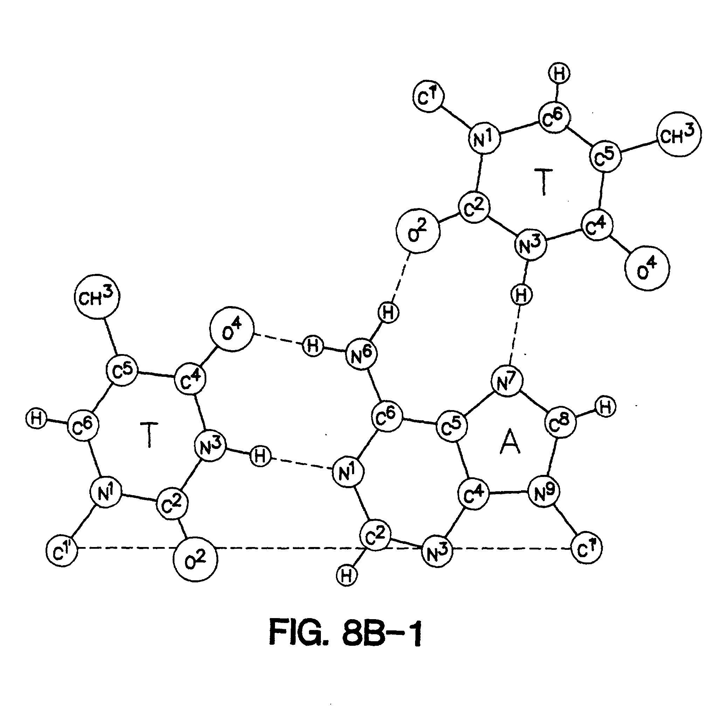

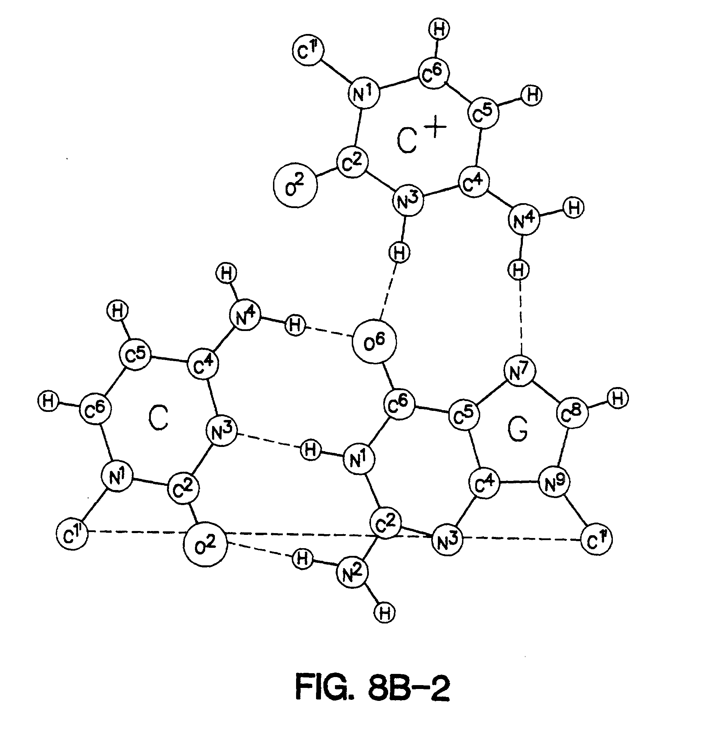

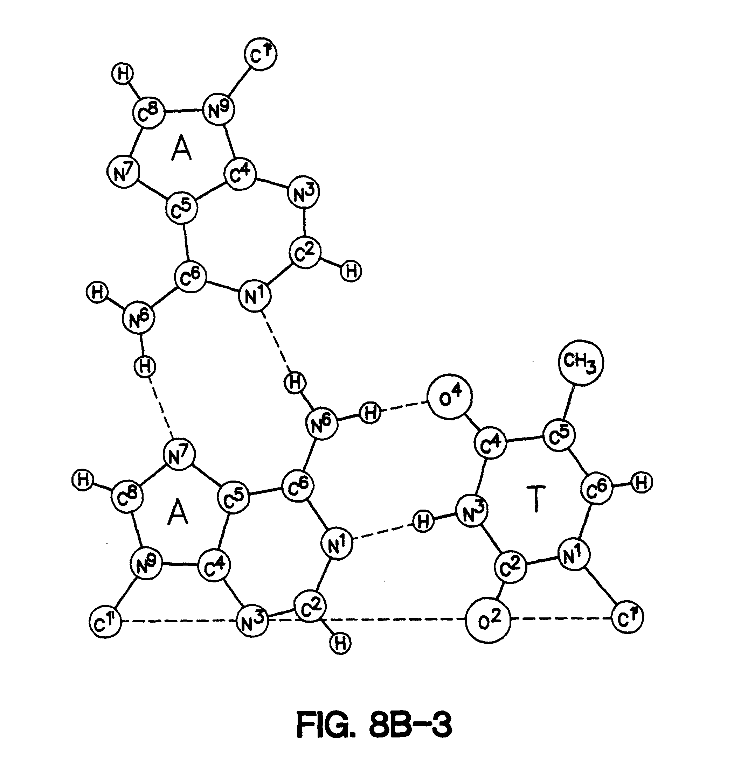

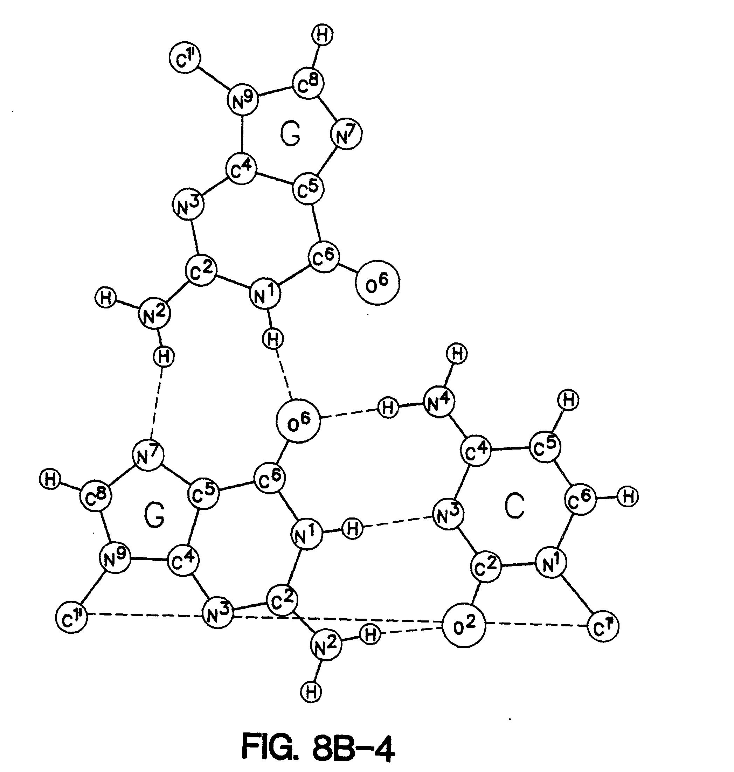

Figure 8 proposes a pairing scheme for a triple helix formed by recombinase proteins. A. Pairing of a third strand to a homologous duplex in the major groove. Duplex strands retain Watson-Crick base pairing shown by solid lines. Non-Watson-Crick pairing involving the third strand and a purine in either the Watson (W) or Crick (C) strand of the duplex is shown by the dotted line. Shaded area represents the phosphate backbone of each strand. Note that the third strand is parallel to the identical Watson strand. B. Proposed hydrogen bonding scheme for all four possible base triplets. Cytosine residues in the third strand are protonated at the $N_3$ position to allow formation of two hydrogen bonds.

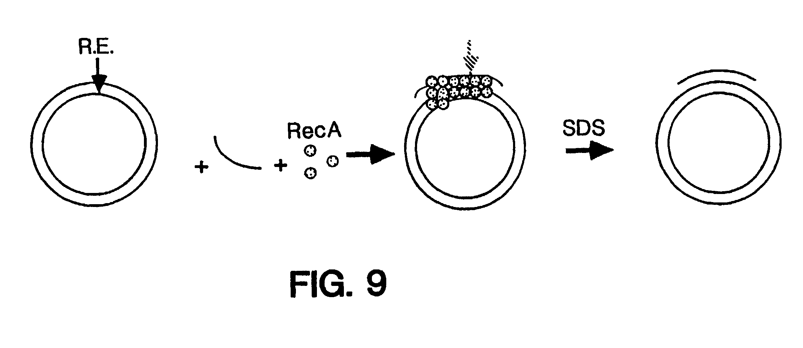

FIGURE 9. Scheme for the formation of synaptic complexes and stable joint molecules. A duplex DNA and a homologous oligonucleotide are incubated in the presence of E. coli recA protein to form a synaptic complex in which the two DNAs are paired within a recA nucleoprotein filament. The formation of synaptic complexes is monitored by the inability of a restriction endonuclease to cleave the duplex within the region of pairing. If recA protein is removed from synaptic complexes by the addition of SDS detergent, stable, deproteinized joint molecules result.

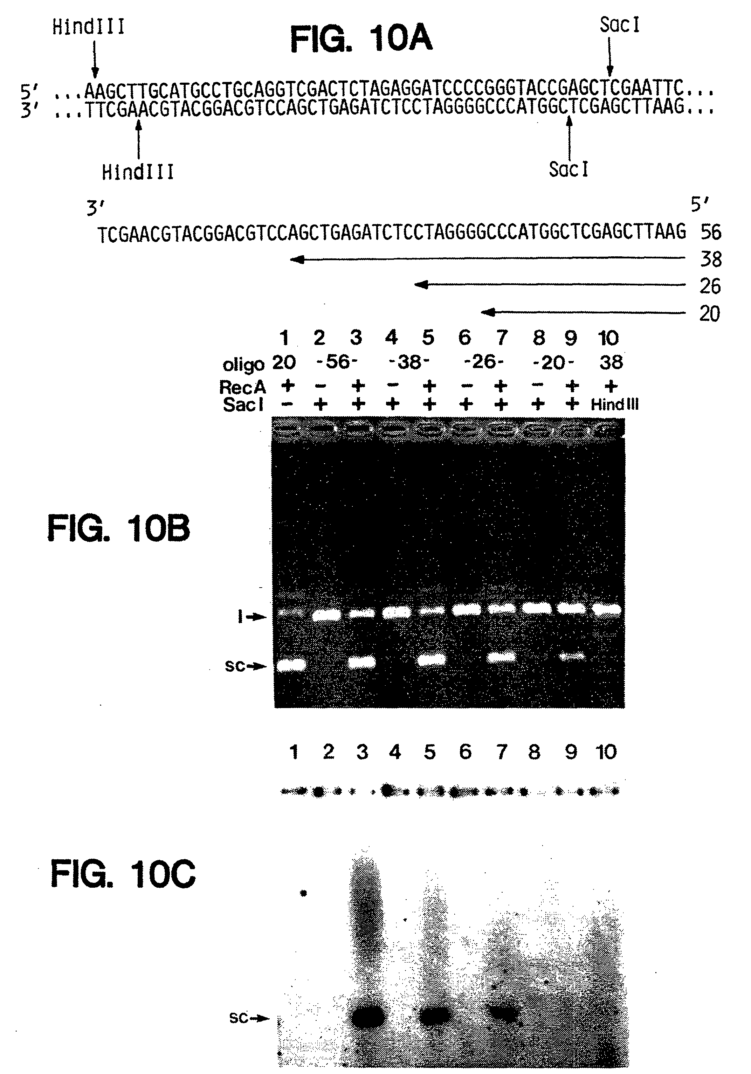

FIGURE 10. Synaptic complexes and joint molecules formed by recA. Fig. 10A. Oligonucleotides having 56, 38, 26 or 20 bases of homology to pUC18 duplex DNA that span a Sac I site. Fig. 10B. Synaptic complexes formed by recA. $^{32}$P-labeled oligonucleotides, pUC 18 supercoiled plasmid DNA and recA protein were co-incubated. Following incubation with the appropriate restriction endonuclease, the reactions were brought to 1% SDS and electrophoresed on a 1% agarose gel containing ethidium bromide. The footprint of synaptic complexes is represented by the presence of supercoiled plasmid DNA remaining after incubation with a restriction endonuclease. Fig. 10C. Joint molecules formed by recA. An autoradiogram of the same agarose gel demonstrates the formation of joint molecules as indicated by the presence of $^{32}$P-label migrating at the position of supercoiled plasmid DNA. 1, linear duplex; sc, supercoiled duplex.

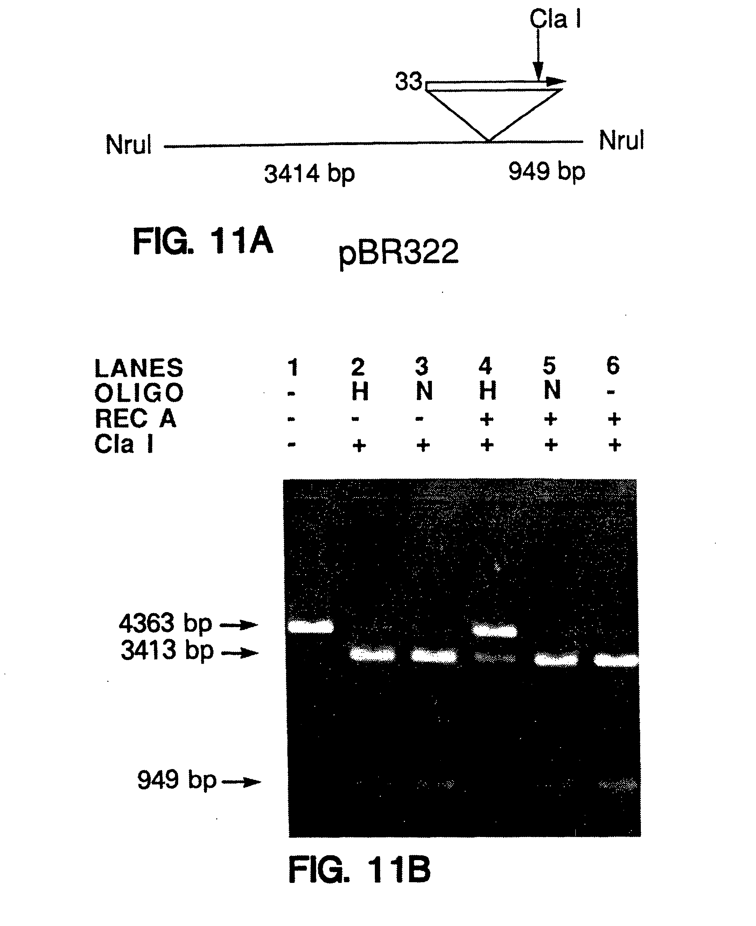

FIGURE 11. Formation of synaptic complexes with linear duplexes. Fig. 11A. Map of the DNA substrates showing the 33-base oligonucleotide homologous to the linear pBR322 duplex and the location of the Cla I site. Fig. 11B. synaptic complex assay with a linear duplex. The nonhomologous oligonucleotide is an M13mp18 sequence corresponding to positions 6228-6260. H, homologous 33-base oligonucleotide; NH, nonhomologous 33-base oligonucleotide.

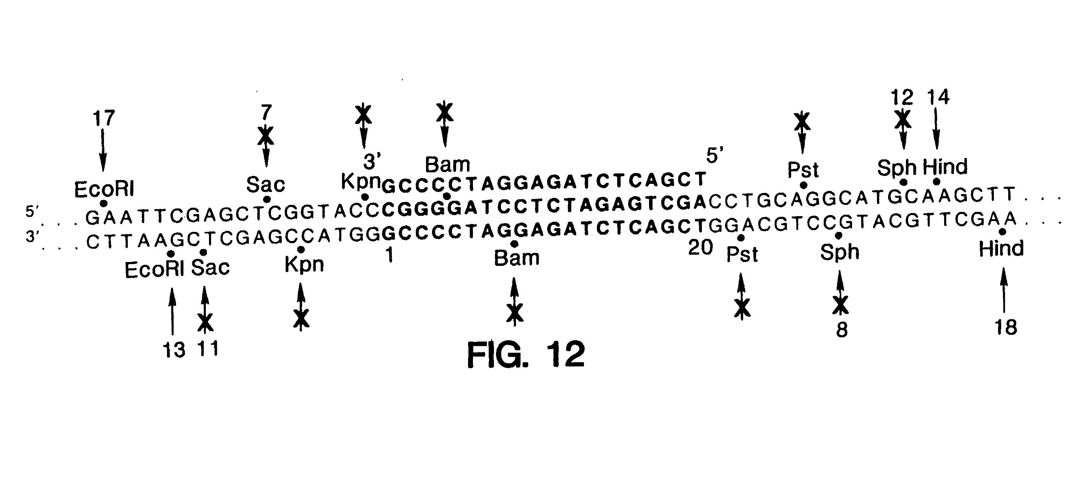

FIGURE 12. Extent of the restriction endonuclease footprint of synaptic complexes. Synaptic complexes were formed with a homologous 20-base oligonucleotide and pUC18 duplex DNA in the presence of recA. The complexes were incubated with a variety of restriction endonucleases whose cleavage sites are indicated by the arrows. Protection from cleavage afforded by the synaptic complex extended to Sac I and Sph I sites. No protection was observed at Eco RI or Hind III sites. Numbers indicate the length from the end of the oligonucleotide to the proximal cleavage site.

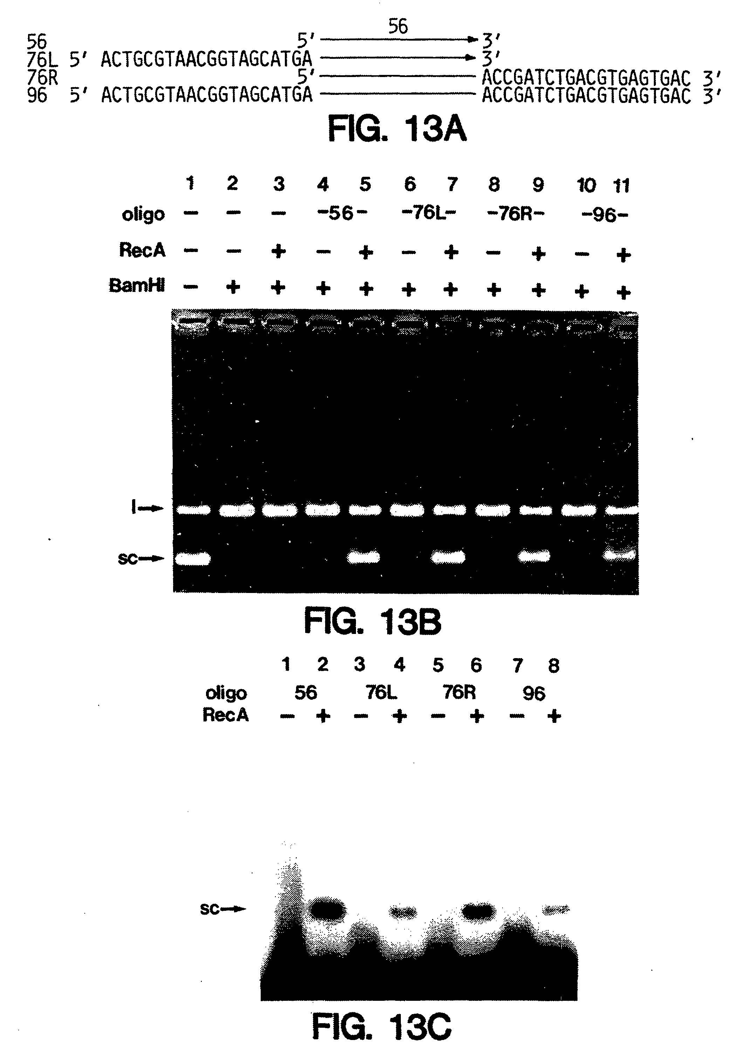

FIGURE 13. The effect of directionality on synaptic complex and joint molecule formation. Fig. 13A. A 56-base oligonucleotide completely homologous to the polylinker region of pUC18 or having additional nonhomologous sequences at either or both the 5' and 3' ends. Fig. 13B. Formation of synaptic complexes by recA can initiate at either the 5' or 3' end of the single-strand or at an internal site. Synaptic complex assays were carried out with $^{32}$P-labeled oligonucleotides. The band in lane 1 migrating just above linear pUC18 represents nicked duplex present in the starting substrate. Fig. 13C. Homology at the 5' end of the single-strand is preferred in joint molecule formation. Synaptic complex assays were deproteinized by the addition of SDS followed by electrophoresis and autoradiography. Lanes 1-8 correspond to lanes 4-11 in part B above. 1, linear duplex; sc, supercoiled duplex.

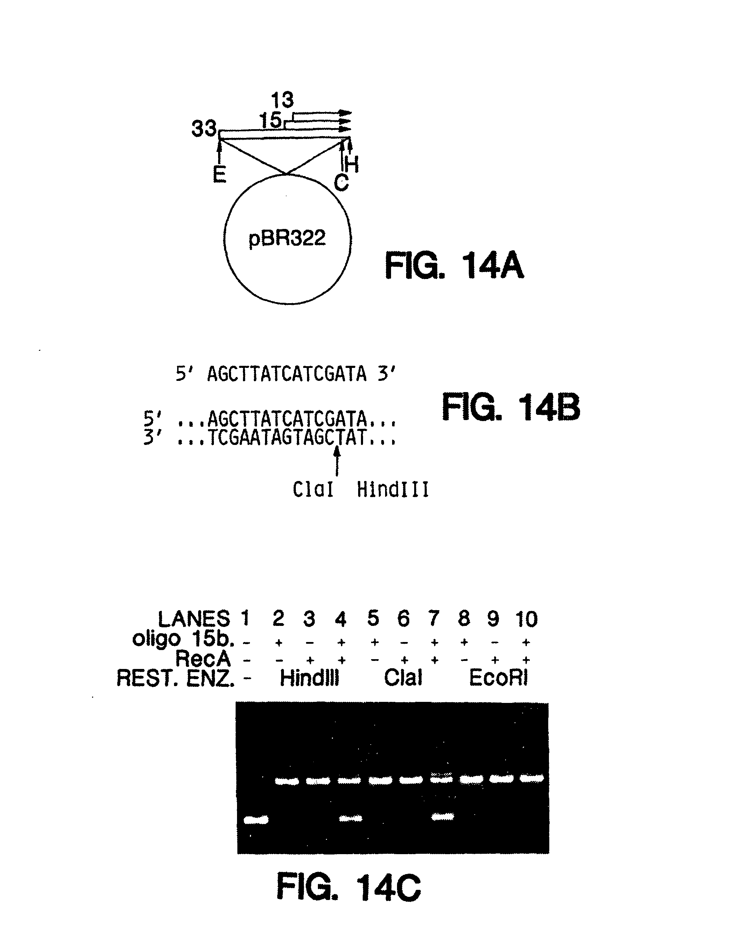

FIGURE 14. The minimal searching unit for homologous pairings. Fig. 14A. Oligonucleotides 33, 15 or 13 bases long are homologous to pBR322. Positions of Eco RI(E), Cla I (C) and Hind III (H) restriction endonuclease sites in the duplex are shown. Fig. 14B. Nucleotide sequence of the 15-base oligonucleotide and corresponding duplex sequence showing positions of Cla I and Hind III cleavage. Bases in the duplex that comprise all or part of the recognition sequence for Cla I and Hind III are indicated in bold. Fig. 14C. Formation of synaptic complexes with an oligonucleotide 15 bases long. Synaptic complexes were formed with the 15-base oligonucleotide as described above. Lanes 2-4, incubation with Hind III; lanes 5-7, incubation with Cla I; lanes 8-10, incubation with Eco RI.

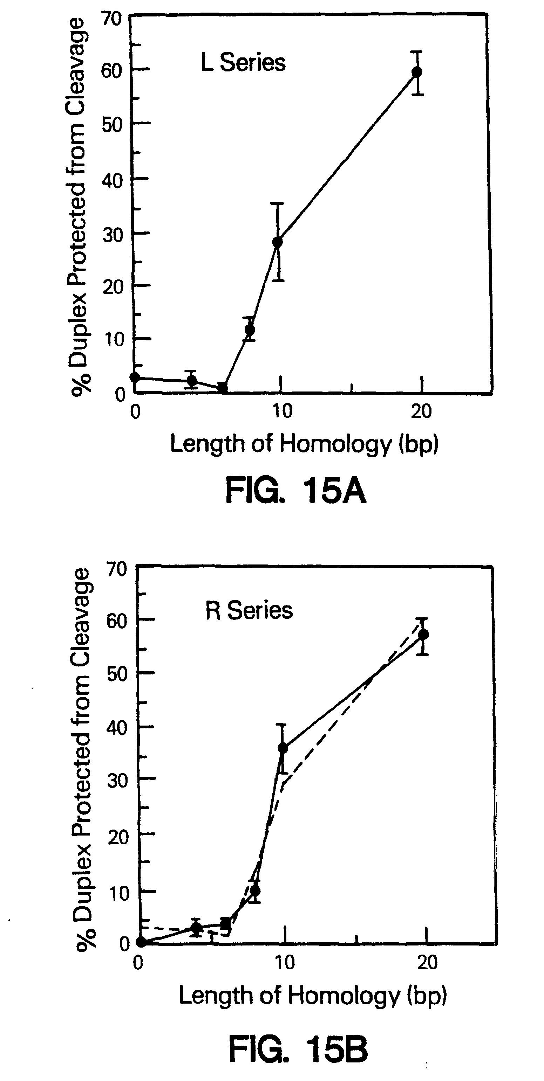

FIGURE 15. RecA pairs less than one helical repeat of the duplex DNA. Fig. 15A. Formation of synaptic complexes with the L series of oligonucleotides. Results represent the average of three independent observations. Percent protection of the duplex is normalized to a control reaction containing duplex DNA and recA. Error bars represent the standard error of the mean. Fig. 15B. Formation of synaptic complexes with the R series (solid line). Results represent the average of four independent observations. Shown for comparison is the corresponding data for the L series, dotted line. The detection and quantitation of small numbers of synaptic complexes was facilitated by the use of 200 ng of duplex DNA in these experiments.

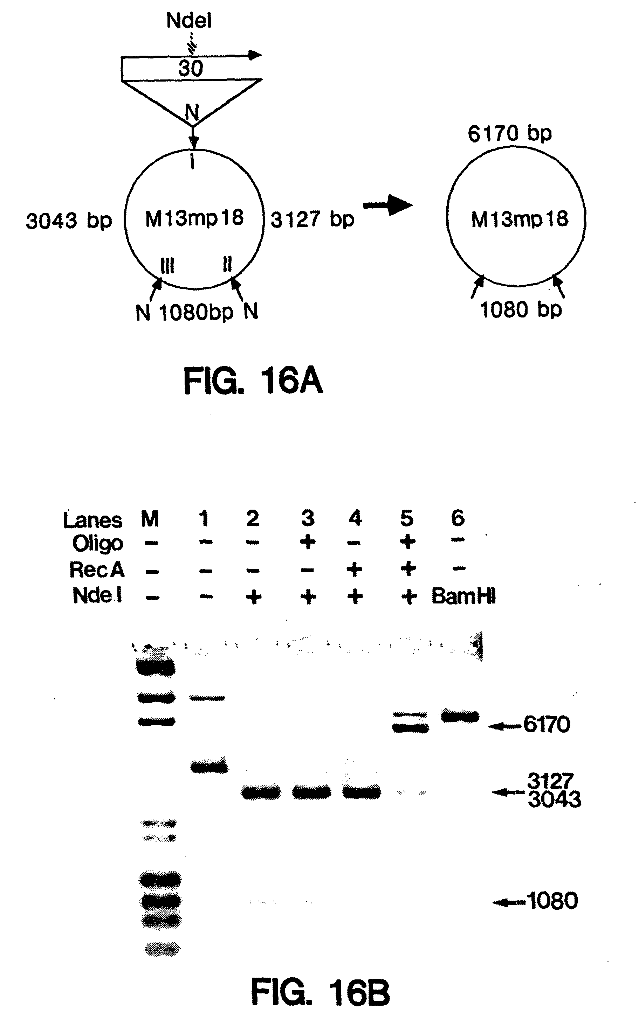

FIGURE 16. The specificity of the recA pairing reaction. Fig. 16A. A 30-base oligonucleotide homologous to M13mp18 spans one of three NdeI restriction endonuclease sites (N) in the duplex. The oligonucleotide sequence is 5'TATCAACCGGGGTACATATGATTGACATGC 3'. The NdeI site is in bold. Fig. 16B. RecA targets synaptic complex formation to the homologous site in the duplex with high efficiency. The 30-base oligonucleotide was incubated with duplex DNA and recA in a synaptic complex assay followed by incubation with NdeI. Size markers (M) are lambda Hind III and phi X 174 Hae III fragments. Lane 6, M13mp18 duplex DNA digested with Bam HI yielding a full-length 7.2 kb linear fragment. For clarity, the ethidium-bromide stained gel is reproduced in reverse contrast.

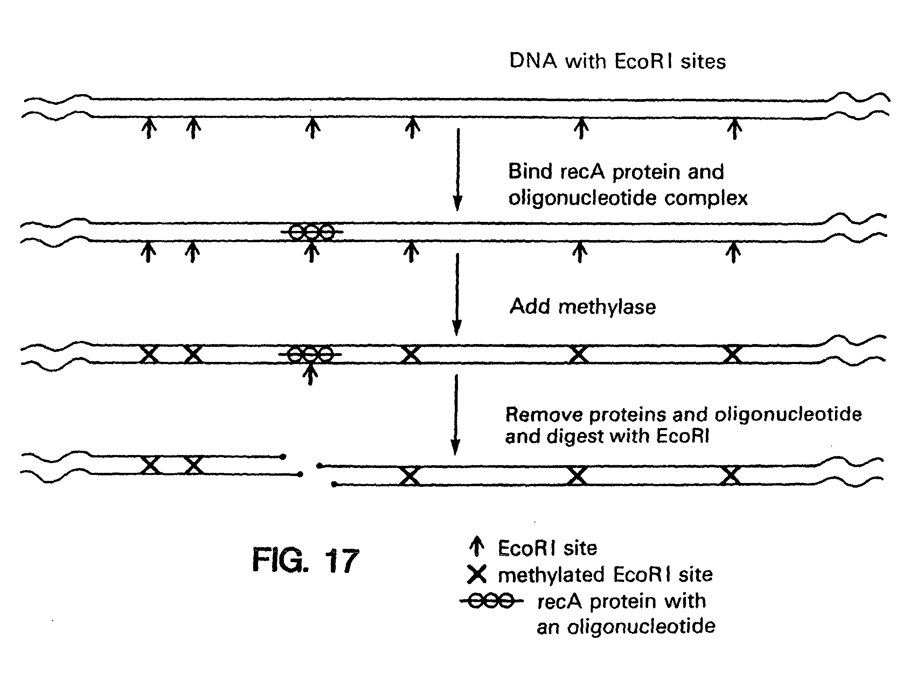

FIGURE 17. Schematic of the strategy used for sequence specific cleavage of DNA. This diagram shows cleavage at a single site.

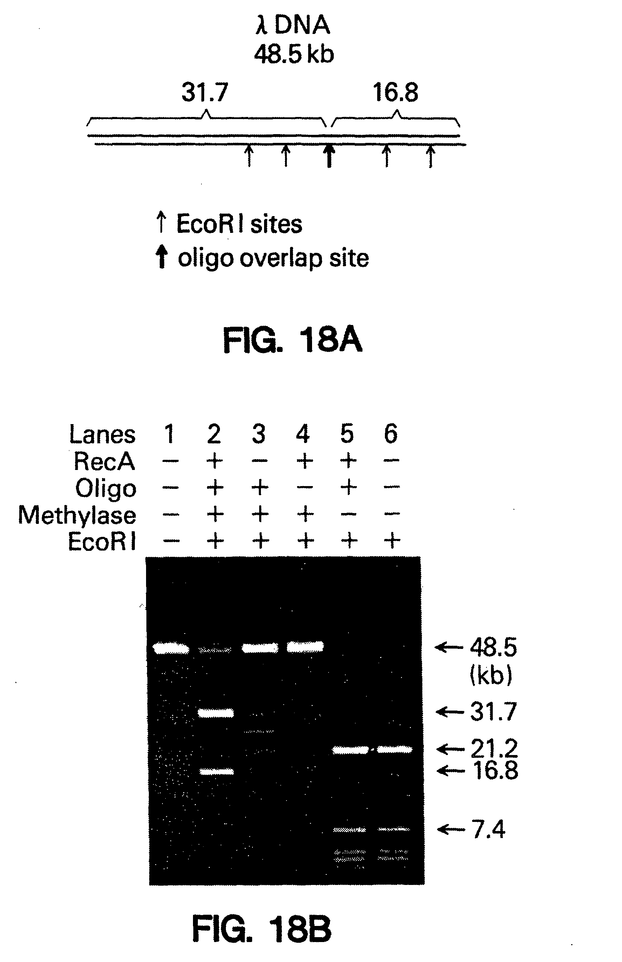

FIGURE 18. Fig. 18A. A schematic showing the position of cleavage of lambda DNA using an oligonucleotide homologous to the site shown by the bold arrow. Lambda DNA contains 5 Eco RI sites, including the one shown by the bold arrow. Fig. 18B. Agarose gel stained with ethidium bromide showing sequence-specific cleavage of lambda DNA. Lane 2 shows the complete cleavage reaction and the other lanes had components omitted as shown. Unmethylated lambda DNA was first protected by incubating with recA protein and an oligonucleotide 30 bases long identical to the lambda sequence from position 31,734 to 31,763. The sequence was 5'-TCACGCCGGAAGTGAATTCAAACAGGGTTC-3'. After 10 minutes at 37°C, a minimal volume of Eco RI methylase and S-adenosylmethionine was added and the reaction was allowed to proceed for 20 minutes. The recA protein and methylase were then inactivated by heating for 15 minutes at 65°C. Eco RI restriction enzyme was added to the tube at 37°C, and the reaction was allowed to proceed for 60 minutes. The reaction volume was 40 µl and contained, in order of addition: 25 mM Tris-acetate (pH 7.5), 4 mM Mg-acetate, 0.4 mM dithiothreitol, 0.5 mM spermidine, 10 µg of recA protein, 100 µM EGTA, 1.1 mM ADP, 0.3 mM ATP-gamma-S (Fluka BioChemica), 0.18 µg of oligonucleotide, 0.9 µg of lambda DNA, 4 µg of acetylated bovine serum albumin (BSA), 3.8 units of Eco RI methylase, 120 µM S-adenosylmethionine, and 20 units of Eco RI restriction enzyme (all reagents listed from lambda DNA on were from New England Biolabs). The Tris-acetate, dithiothreitol, spermidine, and buffers used in the final recA protein purification steps were passed through Chelex 100 (Bio-Rad) columns to remove trace metal contaminants. The reactions were stopped with 5 µl of 6% sodium dodecyl sulfate (SDS), 90 mM EDTA and 0.1% bromophenol blue. 20 µl of the final reaction mixtures were mixed with 60 µl of 0.5% InCert agarose at 65°C and allowed to set in the wells of a 1.5% agarose gel. The gel was run by pulsed field electrophoresis on a CHIEF-DRII system (Bio-Rad) for 36 hours at 12°C, 180 V, and 2.5 s switch time.

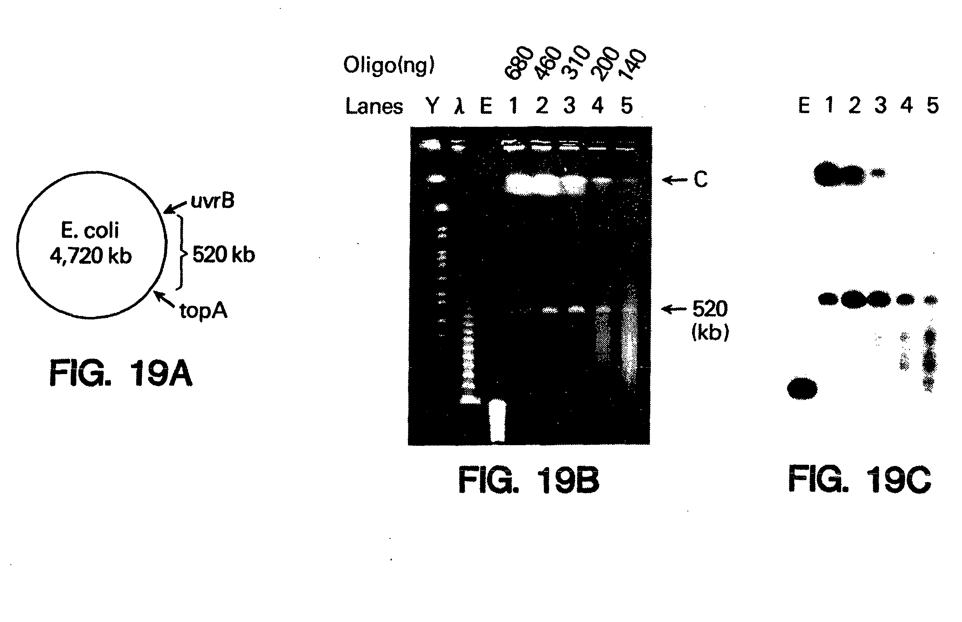

FIGURE 19. Fig. 19A. A schematic showing the positions of cleavage of the E. coli chromosome using two oligonucleotides homologous to sites in the uvrB and topA genes. Fig. 19B. Agarose gel stained with ethidium bromide showing sequence-specific cleavage of E. coli DNA generating a 520 kb fragment. The compression (C) zone of the gel is also shown. Lane Y, yeast S. cerevisiae chromosomal DNA markers. Lane lambda, lambda concatamer DNA ladder. Lane E, unmodified E. coli DNA after a complete digestion by Eco RI. Lanes 1-5, complete cleavage reactions with different amounts of oligonucleotide in each lane. The uvrB oligonucleotide sequence was 5'-TCATGAGTAAACCGTTCAAACTGAATTCCGCTTTTA-3' (36 bases long), and the topA sequence was 5'-CGAGATCGAAGAGGGCGAATTCCGCATTAA-3' (30 bases long). Reaction conditions were similar to those of Figure 18, except the following conditions were modified to obtain good results for agarose-embedded DNA. RecA protein and oligonucleotide were preincubated with the DNA for 15 minutes at 37°C; methylase and S-adenosylmethionine were added and the methylation was allowed to proceed for 1 hour. The methylation was terminated by adding 100 µl of 2% SDS for 30 minutes at 37°C. The beads were then equilibrated in 100 mM Tris-HC1 (pH 8.0), 50 mM NaCl, 1.5 µM dithiothreitol, and 200 µg/ml nonacetylated BSA (Calbiochem-Behring). The observation of Wilson and Hoffman (Wilson et al, *Anal. Biochem*. 191, 370 (1990)), that this buffer is excellent for inhibiting nonspecific or star activity of Eco RI on agarose-embedded DNA, was confirmed. Concentrations of other reagents are as in Figure 18 except that each tube contained 20 µg recA protein, the indicated amount of each oligonucleotide, 30 µl (packed volume) of beads containing E. coli DNA, 40 units of methylase, and digestion was with 40 units of Eco RI restriction enzyme. After stopping the reaction, the beads were run on a 1% agarose gel for 30 hours at 12°C, 160 V, with the switch time ranged from 60-140 s. Fig. 19C. Southern blot of the gel in part (B). The gel was blotted onto a GeneScreen Plus nylon membrane (Dupont) according to the manufacturer's directions. The probe was made by polymerase-chain-reaction amplification (PCR) of a 600 base pair fragment of the trpA gene from E. coli using $^{32}$P-deoxycytidine 5'-triphosphate. The trpA gene lies between the uvrB and the topA gene. The film was overexposed to reveal minor bands, but densitometry was performed on less exposed films. Lane E, which contained the same amount of DNA as lanes 1-5, showed the hybridization of the probe to the predicted 40 kb fragment generated by complete Eco RI digestion (Kohara et al, *Cell* 50, 495 (1987)); the intensity of this band provided the 100% value to calculate the 520 kb fragment yield.

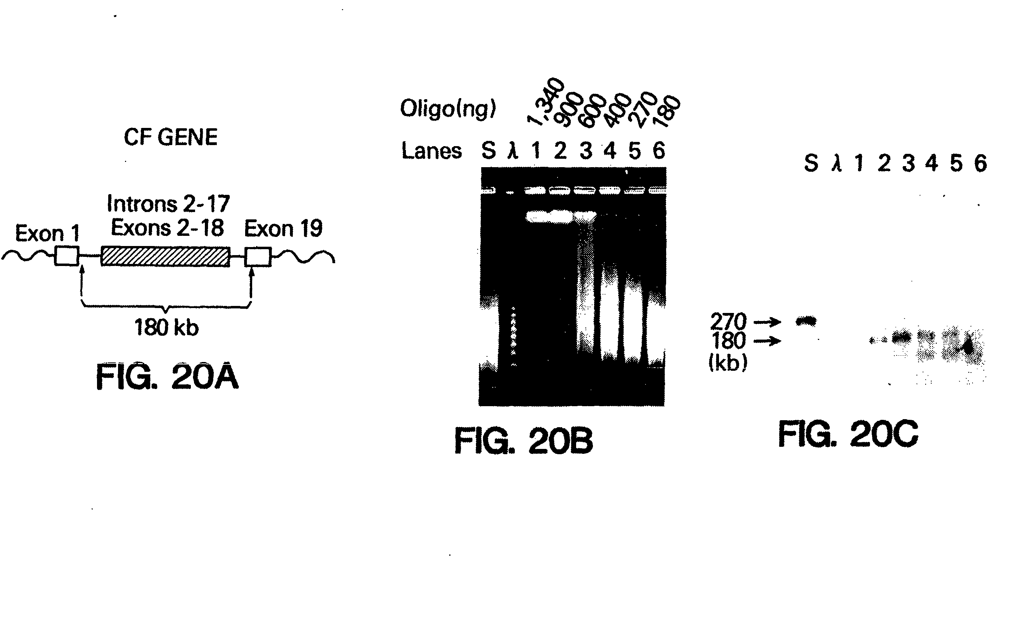

FIGURE 20. Fig. 20A. A schematic showing the positions of cleavage of the human CF locus using two oligonucleotides homologous to sites in intron 1 and exon 19. The gene contains a total of 24 exons. Fig. 20B. Agarose gel stained with ethidium bromide showing development of smaller fragments of DNA as the oligonucleotide concentration was decreased. Lane S, Sfi I digest of unmodified HeLa cell DNA. Lane lambda, lambda concatamer DNA ladder. Lanes 1-6, complete cleavage reactions with the indicated amount of each oligonucleotide. The intron 1 oligonucleotide sequence was 5'-TAAGTGCTCAGAAAACATTTCTTGACTGAATTCAGCCAACAA-AAATTTTGGGGTAGGTAG-3' (60 bases long), and the exon 19 oligonucleotide sequence was 5'-AATGGCCAACTCTCGAAAGTTATGATTATTGAGAATTCACACGTGAAGAAAG ATGACATCTGG-3' (63 bases long). Conditions were identical to Figure 19 except that the reaction volume at all steps was doubled, and 25 µl (packed volume) of HeLa beads were used per reaction. 80 units of Sfi I (New England Biolabs) were used in lane S according to the manufacturer's directions. A 1% agarose gel was run for 32 hours at 12°C, 160 V, with the switch time ramped from 40-120 s. Fig. 20C. Southern blot of the gel in part (B). The Sfi I digest band was 270 kb long and was used in calculating the yield of the 180 kb fragment. The probe was made by PCR of the CF cDNA T8-B3 plasmid (from the American Type Culture Collection). The probe was 550 bases long and contained 410 bases of exon 13 colinear with 140 bases of exon 14.

## DETAILED DESCRIPTION OF THE INVENTION

The present invention relates, to a method of cleaving DNA at any desired site, in which a three-stranded DNA molecule is formed between a DNA duplex and a third DNA strand.

The triplex of the present invention can be formed by contacting a recombinase protein with a DNA duplex and a single stranded DNA sequence under conditions such that hybridization between the duplex and the single stranded molecule is effected. Recombinase proteins include proteins that carry out in vitro biochemical steps that mimic homologous recombination in cells or in vivo and, in particular, that can pair two DNAs, single-stranded or double-stranded, in a homology-dependent manner. Examples of such proteins include procaryotic proteins such as E. coli recA protein and other bacterial recA analogues, and the bacteriophage T4 uvsX protein. Proteins from eukaryotic sources have also been described and are referenced above.

The triplex of the invention is stable even upon removal of the recombinase protein. The complementary strands of the duplex can have any base sequence and the single-stranded molecule can be complementary to as few as 13 bases of the duplex strands for a stable triplex to form. One skilled in the art will appreciate that the minimum number of base pairs of homology required may vary with the recombinase protein used. For example, as few as about 13 bp of homology is recognized by human and Drosophila recombinase, and 38 bp by E. coli recA.

Recombinase protein suitable for use in the present method can be present in purified form or can be partially purified. As noted above, the enzyme can be isolated using methods referenced in the Examples from sources including human cells and cells of Drosophila. Other sources include E. coli. The optimum concentration of recombinase to be utilized can be readily determined by one skilled in the art.

In the method of the invention DNA cleavage is effected in a sequence-specific manner. At present, this technology is restricted by the biological repertoire of available restriction endonucleases. In order to extend the range of sequence specific cleavages, several groups have attempted to direct cleavage to a target site by the use of homopyrimidine oligonucleotides bearing chemical cleavage groups for DNA. The rationale behind this approach is that triplex DNA formation is facile if the third strand consists exclusively of pyrimidines and the target duplex sequence consists of a "complimentary" stretch of homopurine and homopyrimidine strands. Once this three-stranded hybridization is effected, a chemical moiety such as Fe-EDTA or Cu-phenanthroline on the end of the third strand will carry out cleavage of the target duplex DNA. (See, Dreyer et al, 1985, *Proc. Natl. Acad. Sci USA* **82**, 968-972).

The field of sequence specific cleavage is significantly extended by Applicants' discovery that a third strand of any sequence (i.e. one containing both purines and pyrimidines on one strand in any order) can form a triple helix with a homologous duplex sequence in the presence of recombination proteins. Using recombinase proteins to form a triple helix DNA containing a third strand oligonucleotide bearing a chemical cleavage group, (for example Fe-EDTA, Cu-phenanthroline or 2-methoxy-6-chloro-9-aminoacridine), cleavage of any duplex at any given sequence can be achieved. This technology can be expected to allow efficient sequence-specific cleavage of, for example, a 20 base sequence that occurs, for example, only once in the entire mammalian genome. One skilled in the art will appreciate that more frequent cutting of the genome can be achieved by manipulating the stringency of the recombinases for DNA homology (sequence identity shared between the oligonucleotide and the duplex) to accommodate sequences that are similar but not identical, or by directing cleavage to repetitive sequence motifs.

The ability to achieve sequence-specific cleavage at will has several implications. First, it provides an invaluable tool in the molecular cloning of DNA. Second, gene mapping over long stretches of DNA on the order of a million base pairs is rendered relatively straightforward. Third, the ability to introduce a single cut in the mammalian genome, particularly when this is carried out within the cell, greatly facilitates gene targeting.

The invention also relates to a method of using the formation of these three-stranded DNAs in cloning and mapping of DNAs having multiple restriction enzyme cleavage sites. It is oftentimes desireable to cleave at a limited subset of restriction sites. At present, hit and miss "partial cleavage" schemes are used or methylases are used that block cleavage at all sites recognized by certain restriction enzymes. Unfortunately, the repertoire of methylases is extremely limited and to the extent that they are available, result in the modification of all sites recognized by a particular restriction enzyme. Work from several labs has shown that triple helices involving polypurine/polypyrimidine sequences can abrogate cleavage by a restriction enzyme within the region of triple helix DNA. The technology disclosed herein, based on the use of recombinase proteins, can extend such protection from cleavage to virtually any restriction enzyme recognition sequence.

The method to which the invention relates has wide applicability. It can be used to protect a specific sequence (that is, that sequence with which the oligonucleotide is complexed) from modification, for example, by a methylase, or, alternatively, from cleavage, for example, by a restriction enzyme. In addition, the present invention can also be used to effect site specific cleavage by attaching to the oligonucleotide a cleavage moiety, for example, a chemical cleavage moiety. Furthermore, the present method can also be used to accomplish site specific cleavage in a two-step process by, first, protecting the specific sequence from modification (by the formation of the three-stranded molecule) and, then, removing the oligonucleotide, thus making the prior-protected site available for cleavage (the other such sites being protected from cleavage by prior modification). In yet another embodiment, the present invention can be used to enrich a DNA pool for a desired sequence by derivatizing the oligonucleotide with one member of a binding pair, for example, biotin, and then selecting for the three-stranded molecule resulting from the complexation of the oligonucleotide with the desired duplex, using the other member of the binding pair, in this example, avidin. Purification of specific duplex DNA molecules can be accomplished in this manner. Other embodiments of the invention include the use of oligonucleotides linked to detectable labels for tagging specific duplex molecules, and the use of oligonucleotides bound to cross-linking reagents which, when activated, result in the formation of a stable three-stranded molecule. Other applications of the present method will be clear to one skilled in the art from a reading of this disclosure.

Oligonucleotides suitable for use in the invention can be designed so as to optimize desired results. For example, the length of the oligonucleotides can be adjusted for particular targeting protocols without undue experimentation.

While the present invention will be described in some detail with reference to the above-described protection and restriction/modification embodiments, one skilled in the art will appreciate the broader applicability of this methodology both to in _vitro_ and in _vivo_ systems.

The protection aspect of the invention is described in some detail in Example 12 below. As will be clear from a reading of that Example, recA can be used to afford protection, for example, from cleavage, of a particular site in a duplex DNA molecule by effecting the formation of a synaptic complex at that site. Formation of the complex between the oligonucleotide and the homologous duplex DNA is both rapid and efficient.

For purposes of clarity, the specific sequence of reactions involved in the restriction/ modification embodiment of the invention is detailed in Examples 13-15 below. For target DNA that is not easily sheared, such as lambda DNA which is 48.5 kilobases (kb) long (Daniels et al in _Lambda II_, Hendrix et al, Eds. (Cold Spring Harbor, N.Y. 1983), pp. 519-676), the reactions are advantageously done in solution. Larger genomes, however, can be embedded in agarose microbeads. (See Examples 14 and 15.)

The first step in achieving sequence-specific cleavage of duplex DNA, in the exemplified restriction/modification embodiment, is the seiection of the particular Eco RI site for cleavage. A homologous oligonucleotide, generally 30 to 60 bases long, is synthesized such that the Eco RI site is, advantageously, centered in the oligonucleotide. Oligonucleotides having the recognition sequence at the 5' or 3' end can also be used, however, reduced efficiency may be observed (see Example 14). The oligonucleotide and recA protein are incubated with duplex DNA and the complex formed at the site of homology. Eco RI methylase and S-adenosylmethionine are then added and allowed to methylate all available sites, but spare the site involved in the oligonucleotide and recA protein complex. The complex and methylase are then inactivated, and Eco RI restriction enzyme is added to cleave at the now uncovered Eco RI site.

Cutting at a single site is depicted in Figure 17, however, two different oligonucleotides can be added at the same time. This allows isolation of a fragment from long or circular genomes. The following equation describes the yield of such a fragment:

$$\text{Yield (\%)} = (PC)^2[1-(1-M)C]^X(1-N) \times 100$$

where

P is the efficiency of protection by recA protein of homologous sites from methylation (a value of 1 means complete protection),

C is the efficiency of restriction enzyme cleavage,

M is the efficiency of methylation of unprotected sites,

X is the number of Eco RI sites found in the fragment, and

N is the fraction of fragments destroyed by non-specific nucleases or shearing.

The three terms of the equation contain important parameters of the reaction. From the first terms, it is clear that protection of the homologous site should be maximized, and that drops in protection efficiency will be squared when two sites are involved. Protection efficiencies are assumed to be the same for both sites. Because of the second term, which is raised to the X power, the methylation should advantageously, be carried very close to completion, especially for long fragments with multiple internal Eco RI sites. From the third term, it is clear that nonspecific nucleases should be minimized, and, indeed, the use of proteins of high purity is preferred.

One skilled in the art will appreciate from a reading of the foregoing that while the oligonucleotides used can be underivatized, the versatility of targeting can be increased by derivatizing the oligonucleotide. Possible derivatives include proteins, biotin (as noted above), fluorescent dyes, chemically reactive moieties (for example, for cleavage) or photochemically reactive moieties. Derivatization can be effected using methods known in the art.

The method to which the invention relates has many applications, genomic mapping being one. The physical distance between two loci is simply the fragment size. Once a fragment is isolated, for example, using pulsed field gel electrophoresis, the complexity of finding a particular desired gene is reduced several orders of magnitude as compared to working with unfractionated genomic DNA.

One skilled in the art will appreciate from the foregoing that oligonucleotides can be designed to target any site of a DNA sequence, including sites within large genomes. Accordingly, oligonucleotides can be designed which can be used in an intracellular milieu to target cleavage, recombination or repression of specific genes.

Certain aspects of the invention are described in greater detail in the non-limiting Examples that follow.

## EXAMPLES

Experimental details relating to Examples 1-5.

Recombinase Proteins:

Partially purified HeLa recombinase fraction was prepared from nuclear extracts as described elsewhere (Hsieh and Camerini-Otero, 1989, J. Biol. Chem. **264**, 5089-5097). Partially purified Drosophila recombinase, fraction IV, was prepared from 24 hr embryos as described previously (Eisen and Camerini-Otero, 1988, Proc. Natl. Acad. Sci. USA **85**, 7481-7485). The human recombinase preparation was free of any 3'-5' exonuclease activities as determined by trichloroacetic acid precipitable counts. No $^{32}$P cpm were released from $^{32}$P-3' end-labeled duplex DNAs after incubation with human recombinase fraction using joint molecule assay conditions (data not shown). As previously described, the Drosophila recombinase fraction removed <10% of $^{32}$P cpm in a similar assay (Eisen and Camerini-Otero, 1988, Proc. Natl. Acad. Sci. USA **85**, 7481-7485). Although not relevant to the joint molecule assays described here, 5'-3' exonuclease activity in recombinase fraction preparations were exceedingly low; <5% $^{32}$P cpm and 10-15% $^{32}$P cpm were released as trichloroacetic acid soluble counts by the Drosophila and human recombinase fractions, respectively (Eisen and Camerini-Otero, 1988, Proc. Natl. Acad. Sci. USA **85**, 7481-7485). Purified E. coli recA and SSB proteins were generously provided by Dr. Stephen C. Kowalczykowski, Northwestern University Medical School (Kowalczykowski and Krupp, 1987, J. Mol. Biol. **193,** 97-113). Both E. coli proteins migrate as a single homogeneous polypeptide by SDS PAGE and contain no detectable exonuclease activities as judged by release of trichloroacetic acid soluble counts (S. Kowalczykowski, personal communication and data not shown).

DNA Substrates

pGem 4 plasmid DNA was obtained from Promega Biotec. M13mp18 viral DNA and restriction enzymes were obtained from New England Biolabs. M13mp19 viral DNA and pdel 9 plasmid DNA, a derivative of pBR322 deleted from nucleotides 1745 to 2505 (Brenner et al., 1985, Mol. Cell. Biol. **5**, 684-691), were prepared according to standard procedures. Oligonucleotides were synthesized and purified as described elsewhere (Hsieh and Camerini-Otero, 1989, J. Biol. Chem. **264**, 5089-5097). Oligonucleotides homologous to the plus strand of pdel 9 spanned pBR322 positions 17-29, 10-29 and 4359-29 (Sutcliffe, 1978, Cold Spring Harbor Symp. Quant. Biol. **43**, 77-90) for the 13mer, 20mer and 33mer, respectively. Oligonucleotides homologous to the polylinker region of pGem 4 were derived from the negative strand of M13mp19 and mapped to M13mp19 positions listed below (Yannisch-Perron, et al. 1985, Gene **33**, 103-119). Oligonucleotides used for partial duplexes spanned positions 6278-6290, 6265-6290, 6253-6290 and 6235-6290 for 13 bp, 26 bp, 38 bp and 56 bp respectively; those used for branch migration structures spanned 6278-6300, 6265-6300, 6253-6300 and 6235- 6300 for 23bp, 36 bp, 48 bp and 66 bp, respectively. Oligonucleotides were labeled with $^{32}$P-gamma-ATP (New England Nuclear) and T4 polynucleotide kinase (Pharmacia) and de-salted by passage over G25 spin columns (Boehringer Mannheim) or by dialysis. Preparation of $^{32}$P-labeled partial duplexes from labeled oligonucleotides and M13mp19 viral strand DNA was as described elsewhere (Hsieh and Camerini-Otero, 1989, J. Biol. Chem. 264, 5809-5097). The branch migration structure was formed by incubating the partial duplex molecule with an unlabeled oligonucleotide identical in sequence to the $^{32}$P-labeled annealed oligonucleotide, but containing an additional 10-base annealing site immediately 5' to the $^{32}$P-labeled fragment. DNA concentrations are expressed as moles of nucleotides or by weight.

The length of homology shared between a single-strand and double-strand substrate is defined as the maximum number of bases that could be paired between the single-strand DNA and the complementary strand of the linear duplex substrate in a joint molecule. Two significant regions of homology shared between pGem 4 and M13mp18 were found using the Univ. of Wisconsin BESTFIT program. They are in opposite orientations with respect to each other in pGem 4. Map positions for the 59 bp region from the lac i gene are: M13mp18 6001-6059 (Yannisch- Perron et al., 1985, Gene33, 103-119) and pGem 4 104-162. The 57 bp polylinker region maps to M13mp18 positions 6231-6287 and in pGem 4 to positions 10-66. pGem 4 Linear Duplex with a Unique 3' $^{32}$P-label

pGem 4 DNA was linearized with Hind III and labeled by filling in 3' ends with $^{32}$P-alpha-dATP (New England Nuclear) and cold deoxynucleotides using the Klenow fragment of DNA polymerase (Pharmacia). The reaction was stopped by heating at 65°C for 10 min, was brought to 100 mM NaCl and digested with Bgl I. The 1,635 bp Bgl I-Hind III fragment containing the polylinker region was gel purified and dialyzed extensively against 10mM Tris-HCl, pH 8.0 and 1mM EDTA. When this $^{32}$P- labeled fragment was digested with Pst I and analyzed by densitometry after electrophoresis on an 8% denaturing polyacrylamide gel or a 1% agarose gel, >99% of the label was removed from the 1.6 kb fragment. The $^{32}$P- label thus resided within 10 bases of the 3' end of the plus strand of the duplex substrate.

Joint Molecule Assays

Joint molecule assays, 20 ul, were carried out for 10 min at 37°C as described (Hsieh and Camerini-Otero, 1989, J. Biol. Chem. **264**, 5089-5097) using 150 pmol (50 ng) of M13mp18 viral DNA and 150 pmol (50 ng) linear pGem 4 DNA or 300 pmol (100 ng) linear pdel 9 and 5 ng $^{32}$P- labeled oligonucleotide with 100 ng HeLa protein or 40 ng Drosophila protein. Assays containing 7 ug recA protein and 700 ng SSB protein were carried out for 10 min at 37°C with preincubation as described (Hsieh and Camerini-Otero, 1989, J. Biol. Chem. **264**, 5089-5097). Assays were brought to 1% SDS and 10 mM EDTA prior to electrophoresis at room temperature on 0.7% agarose gels in TAE buffer (40 mM Tris-acetate, pH 8, 1 mM EDTA) containing ethidium bromide for 12-16 hr at 0.6V/cm. Quantitation was accomplished by densitometry. Electron microscopy was performed using the modified Kleinschmidt technique.

Thermal Stability Assays

$^{32}$P-labeled partial duplexes (150 pmol) consisting of a $^{32}$P-labeled oligonucleotide annealed to M13mp19 viral strand DNA were incubated for 10 min at the indicated temperatures or on ice in strand exchange buffer containing 1% SDS and 10 mM EDTA. TAE buffer containing 0.1% bromophenol blue and 50% glycerol was added and the mixtures were electrophoresed for 12- 16 hr at 0.6V/cm on 0.7% agarose gels in TAE buffer containing ethidium bromide followed by autoradiography. For stability assays of branch migration structures, 150 pmol of the $^{32}$P-labeled partial duplexes and 4 ng of a second oligonucleotide containing a 10 base annealing site to M13mp19 were incubated for 10 min at the indicated temperature or on ice in strand exchange buffer containing 1% SDS, 10 mM EDTA and 10% w/v polyethylene glycol. The reactions were electrophoresed on agarose gels followed by autoradiography as described above. Joint molecules were formed by recombinases. The reactions were stopped in 1% SDS and 10mM EDTA, incubated an additional 10 min at the indicated temperatures or on ice and electrophoresed on agarose gels.

Deproteinizing Joint Molecules

Reacted joint molecule assays containing recA and SSB proteins were brought to 1% SDS, 10mM EDTA and 20 ug/ml Proteinase K (Boehringer Mannheim) and incubated for an additional 20 min at 37°C. The assays were extracted sequentially with phenol, phenol-chloroform and chloroform. The samples were extracted four times with anhydrous ethyl ether saturated with water just before use. Residual ether was removed under nitrogen. The deproteinized joint molecules were analyzed by SDS PAGE (Laemmli, 1970) on 12% polyacrylamide gels (Novex) followed by silver staining (Hochstrasser et al., 1988, AnalBiochem **173**, 412-423) or Western blotting. Electrophoresis and transfer to nitrocellulose using a Schleicher and Schuell Miniblot apparatus were according to manufacturer's directions. Following transfer, the nitrocellulose filters were blocked in 10% milk in phosphate buffered saline, PBS, for 1 hr. The filters were incubated for 1 hr with 20 ml of a 1:500 dilution of rabbit antisera raised to purified recA protein (Hazleton Biotechnology) in 5% milk, 0.1% v/v Tween 20, in PBS. After extensive washing, the filters were incubated for 1 hr with 20 ml of 5% milk, 0.1% Tween 20 in PBS containing 15 µCi of $^{125}$I- labeled donkey anti-rabbit IgG (3000 Ci/mmol, Amersham) followed by extensive washing. Autoradiography was for 3 days using intensifying screens.

Example 1. **Joint Molecyles with Short Regions of Homology**

To test for the formation of three-stranded DNA, recombinase proteins were monitored for their ability to form stable joint molecules with short regions of homology. Recombinase proteins were incubated with a linear duplex DNA and a homologous circular single-strand DNA to form a joint molecule product in which the two substrates are non-covalently joined. Stable joint molecule formation initiates from the ends of the linear duplex. The circular single-strand DNA substrate is the viral (plus) strand of M13mp18 phage. The linear duplex substrate is a plasmid, pGem 4 (Fig. 2A). pGem 4 and M13mp18 share two significant regions of homology, a 59 bp region from the E. coli lac i gene and a homologous 57 bp region that constitutes the polylinker cloning sites of both pGem 4 and M13mp18. These two homologous regions are separated in pGem 4 by 37 bp of nonhomologous sequence. When pGem 4 is linearized in the polylinker region, the polarity of strand exchange by the human (HeLa) and Drosophila recombinase fractions dictates that only homologous sequences at the right end of the pGem 4 linear duplex as shown in Fig. 2A are utilized (Hsieh et al., 1986, Cell **44,** 885-894; Eisen and Camerini-Otero, 1988, Proc. Natl. Acad. Sci. USA **85**,7481-7485). The right end of pGem 4 as drawn exposes the 3' end of the plus strand of the M13mp18 polylinker sequence.

HeLa recombinase fraction formed joint molecules between M13mp18 single-strand DNA and linearized pGem 4 double-strand DNA (Fig. 2B). The samples were deproteinized prior to electrophoresis. Surprisingly, as few as 13 bases of shared homology was sufficient for the formation of stable joint molecules (lane 4). Homologous sequences five bases long on the right end of the linear duplex and 52 bases long on the left end of the duplex were not utilized (lane 5). This result confirms the directionality of strand exchange by the human recombinase fraction. Control experiments using M13mp19 single- strand DNA containing the negative strand of the polylinker region resulted in the appearance of stable joint molecules when pGem 4 was linearized with Eco RI but not Hind III. Deletion of all but 5 bp of homology in assays using pGem 4 digested with both Hind III and Eco RI (lane 6) also yielded no product indicating that the second region of shared homology located at an internal site 37 bp from the left end of the duplex is not utilized by the HeLa recombinase fraction in forming joint molecules. The joint molecule assay using very short sequence homologies was surprisingly efficient with 16-26% of the duplex converted to joint molecules. Under similar assay conditions using completely homologous DNAs, human recombinase fraction converts one-third to one-half of the linear duplex to joint molecules (Hsieh, et al., 1986, Cell **44**, 885-894; Hsieh and Camerini-Otero, 1989, J. Biol. Chem. **264**, 5089-5097).

Like the human recombinase fraction, the Drosophila recombinase fraction also formed joint molecules with as few as 13 bp of homology (Fig. 2C lane 4), did not utilize 5 bp of homology and exhibited the expected directionality of joint molecule formation (lane 5). Approximately 20% of the linear duplex was converted to joint molecules in lanes 1-3. Under similar assay conditions using completely homologous DNAs, Drosophila recombinase fraction converts two-thirds of the duplex to joint molecules (Eisen and Camerini-Otero, 1988, Proc. Natl. Acad. Sci. USA **85**, 7481-7485).

Joint molecule assays using pGem 4 linear duplex, M13mp18 single-strand DNA, recA protein and E. coli SSB protein are shown in Fig. 2D. Assay conditions were those that have been shown by others to promote extensive strand exchange by recA over thousands of basepairs (reviewed in Radding, 1982 Ann. Review Genetics **16** 405-437). It was observed that 56 or 38 bp of homology (lanes 1 and 2) was sufficient for the formation of joint molecules by recA; 28% and 19% of the linear duplexes were converted to joint molecules,. respectively. However, no joint molecules were observed when the homology was limited to 26 bp (lane 3). Under identical reaction conditions using completely homologous substrates, recA was observed to convert essentially all of the duplex to higher order structures. In this assay, the directionality of recA was identical to that of the eukaryotic recombinase fractions, i.e., only homology on the right end of the linear duplex was utilized by recA protein (compare lanes 1 and 5). As observed for the human recombinase fraction, joint molecules were formed efficiently with Eco RI-linearized pGem 4 but not with Hind III-linearized duplex when recA and SSB proteins were incubated with M13mp19 single-strand DNA. At present, it is unknown why the polarity of strand pairing by recA with short regions of homology appears different from that observed by others with substrates sharing extensive regions of homology (reviewed in Radding, 1982). It has been observed, however, that the directionality of strand exchange by recA, unlike that of the eukaryotic recombinases, is substrate-dependent (Konforti and Davis, 1990, J. Biol. Chem. **265**, 6916-6920).

Electron microscopy of joint molecules formed by recA confirmed that the pairing of a linear duplex to one single-strand circular DNA occurred at the end of the duplex. Although no displaced strand was seen, the resolution was insufficient for determining the configuration of the third strand in the region of pairing. In addition, the formation of joint molecules by all three recombinases required the simultaneous presence of both DNAS and recombinase protein and was not due to exonucleolytic processing of the duplex and subsequent annealing of the single-strand DNA (see Fig. 6). When M13mp18 single-strand DNA and Hind III-linearized pGem 4 DNA (56 bp of homology) were separately incubated with recombinase followed by co-incubation of the treated substrates at 65°C for 10 min in the presence of 1% SDS and 10% w/v polyethylene glycol to promote nonenzymatic annealing no joint molecules were formed.

Example 2. **A Novel Joint Molecule Assay**

An explanation for the stability of these joint molecules is that the circular single strand is nonspecifically held in place or "pinched" by the duplex at the junction of the region of homology and nonhomology. To rule this and other forms of trapping out, a novel assay was designed in which the single strand has two ends, is very short and is completely homologous to the duplex. Furthermore, this assay demonstrates that recognition of short homologies is not limited to the polylinker sequence of M13mp18. A novel assay for the formation of joint molecules between a linear duplex DNA and a 5'-$^{32}$P-labeled oligonucleotide identical to the 3' end of one strand of the linear duplex is shown in Fig. 3A. The formation of stable joint molecules was monitored by the appearance of label migrating at the position of linear duplex DNA on agarose gels.

Stable joint molecules were formed by the human recombinase fraction between pdel 9 linear duplex DNA and an oligonucleotide 33 bases or 20 bases in length (Fig. 3B, lanes 1 and 3). No joint molecules were formed with an oligonucleotide 13 bases long (lane 5) or with a nonhomologous oligonucleotide. Approximately 50% of the linear duplex in lane 1 was converted to joint molecules. Control experiments in lanes 2, 4 and 6, demonstrate that joint molecules were not formed by degradation of the duplex by potential exonucleases in the recombinase fraction preparations thereby exposing the duplex strand that could anneal to the oligonucleotide; when the two DNA substrates were separately incubated with recombinase fraction and then co-incubated for 10 min at 65°C in the presence of SDS to stop activity, virtually no joint molecules were observed. No joint molecules were formed when an oligonucleotide corresponding to the negative strand of pdel 9 was used, reflecting the 3'-5' directionality of the human recombinase fraction, i.e. pairing of the oligonucleotide initiates at the 3' end of the noncomplementary strand of the duplex.

Example 3. **Thermal Stability of Joint Molecules**

If the deproteinized joint molecules with short regions of homology discussed above had a displaced strand, branch migration would lead to their rapid dissociation. DNA branch migration is a random walk process where, depending on the geometries of the DNAs examined, the time required to step through a base pair is between 12-200 microseconds (Thompson, et al., 1976, Proc. Natl. Acad. Sci. USA **73**, 2299-2303; Radding, et al. 1977, J. Mol. Biol. **116**, 825-839; Green and Tibbetts, 1981, Nucl. Acids Res **9**, 1905-1918). Thus, the time required to reach the end of the duplex is approximately equal to the distance (56 bp) squared multiplied by the step time (0.2 msec) divided by 2 or about 300 milliseconds (Feller, 1957, Vol. 1. p. 325, John Wiley & Sons, New York). Surprisingly, these deproteinized joint molecules were observed to be very stable for hours (>$10^4$ sec) at room temperature.

The stability of joint molecules at room temperature reflects the formation of additional interactions between the third strand and the duplex. In order to ascertain the strength of these interactions and to rule out covalent interactions, the thermal stability of joint molecules was examined and compared with the stabilities of partially duplex molecules and molecules that can undergo non-enzymatic branch migration (see Fig. 4A). The short duplex regions of the partially duplex molecule, I, corresponded exactly in sequence and length to the presumed paired regions of joint molecules formed by recombinase between pGem 4 linear duplexes digested with Hind III (56 bp), Sal I (38 bp), Bam HI (26 bp) and Kpn I (13 bp) and M13mp18 single- strand DNA shown in Figure 2. The branch migration structure, II, corresponded exactly in sequence, length and direction of potential branch migration to the joint molecules formed by recombinases (see Materials). Non-enzymatic branch migration would result in the displacement of the $^{32}$P-labeled oligonucleotide. In this assay, displacement of the annealed fragment was via homology-dependent branch migration since no displacement of a $^{32}$P-labeled fragment was observed when the second oligonucleotide contained a 10 base annealing site attached to a sequence not homologous to the duplex region.

Representative data from stability experiments for a homologous region of 38 bp corresponding to joint molecules formed between Sal I-linearized pGem 4 and M13mp18 DNAs are shown in Fig. 4B. While the duplexes (58% G-C) were stable after 10 min at 65° C, melting at 75°C, branch migration structures dissociated after 10 min at 45°C. Joint molecules formed by human recombinase fraction were stable after 10 min at 65°C and dissociated at 75°C. This represents a 30°C difference between the relative stability of joint molecule products formed by recombinase versus branch migration structures of identical length, sequence and direction of branch migration. The faint band migrating slower than joint molecules in the ethidium bromide-stained gel is a dimer of pGem 4 resulting from a contaminating ligase activity.

Similar thermal stability data for duplexes, branch migration structures and joint molecules formed by the three recombinases over four different lengths of homology are summarized in Fig. 5. Joint molecules were exceedingly stable dissociating at temperatures 20 to 30 degrees higher than those of branch migration structures. In all cases, observed melting temperatures of the duplexes were in close agreement with calculated $T_m$'s based on sequence length and G-C content (Sambrook, et al., 1989 Molecular Cloning: a Laboratory Manual. Cold Spring Harbor Press, Cold Spring Harbor). In addition, the observation that dissociation temperatures for joint molecules are proportional to the length of homology makes it highly unlikely that a covalent bond is responsible for the stability.

Joint molecules were not stabilized by nonspecific trapping or pinching of the DNA substrates resulting from the use of a large, circular single-strand DNA, e.g. a joint molecule having a structural block right at the exchange point flanked by normal duplex DNA (see also Fig. 3). Joint molecules formed by human recombinase fraction between an M13mp18 linear duplex and a homologous $^{32}$P-labeled oligonucleotide 26 bases long were also significantly more stable than corresponding branch migration structures. Deproteinized joint molecules dissociated at 55° C as did the corresponding duplexes (42% G-C) while branch migration structures were unstable at room temperature (data not shown).

Reconstruction of joint molecule structures nonenzymatically was performed in order to assess their stability relative to joint molecules formed by recombinases. Heat-denatured linear pGem 4 and M13mp18 single-strand DNA were annealed at neutral pH or at pH 5.5 (Voloshin, et al., 1988, Nature **333**, 475-476) in the presence of 10% w/v polyethylene glycol and electrophoresed on agarose gels. No joint molecules were observed. Similarly, annealing heat-denatured linear pdel 9 with $^{32}$P-labeled oligonucleotides failed to yield stable joint molecules.

Example 4. **Joint Molecules That Are Stable Have Three Intact Strands**

The surprising stability of these joint molecules could be explained if the 3' end of the plus strand of the linear duplex had been exonucleolytically degraded leaving only heteroduplex DNA in the joint molecule (see Fig. 1, top). The following experiment shows that stable joint molecules have an intact third strand (the plus strand of the polylinker region).

Joint molecule assays using HeLa recombinase fraction or recA and SSB proteins were carried out between M13mp18 single-strand DNA and a fragment of pGem 4 $^{32}$P-labeled at the extreme 3' end of the plus strand of the linear duplex (Fig. 6A). Restriction enzyme digests of the labeled duplexes confirmed that >99% of the $^{32}$P-label was restricted to the 3' terminal 10 bases of the plus strand of the pGem 4 fragment (see Methods). Thus, formation of $^{32}$P-labeled joint molecules would indicate that the third strand is intact since heteroduplexes of 9 bp or less are very unstable and impossible to recover under these conditions (cf. Fig. 5). Since 15-20% of the labeled duplex was converted to joint molecules in these assays, virtually all of the $^{32}$P-labeled joint molecules must have intact third strands. The thermal stability of $^{32}$P-labeled joint molecules formed by recA (Fig. 6B) indicated that molecules having three intact strands dissociated at relatively high temperatures (cf. Fig. 5). The rapidly migrating lower band represents the nonenzymatic loss of $^{32}$P-label from the $^{32}$P-labeled duplex which occurred at higher temperatures in the absence of recombinase protein.

Quantitation of the relative stabilities of $^{32}$P-labeled joint molecules formed by recA and HeLa recombinase fraction and the corresponding 56 base branch migration structure is shown in Fig. 6C. The $^{32}$P-labeled joint molecules formed by human recombinase fraction, like those formed by recA protein, were exceedingly stable. These results using a truncated pGem 4 linear duplex also demonstrate that the stability of joint molecules derives from pairing restricted solely to the region of homology in the polylinker at the right end of the linear duplex.

Example 5. **Stable Joint Molecules Are Devoid of Protein**

A possible explanation for the stability of joint molecules is that they remain associated with recombinase protein in the presence of 1% SDS. The example depicted in Fig. 7, demonstrates that the stable DNA joint molecules formed by recA protein are devoid of any associated recA molecules after treatment with several deproteinizing agents. Joint molecules having 56 bp of homology were deproteinized with Proteinase K in the presence of 1% SDS and extracted with phenol/chloroform as described above. The level of residual recA protein was assessed by Western blotting using a polyclonal antibody against recA protein (Fig. 7A). Identically treated joint molecule were also analyzed for thermal stability and dissociated between 75° and 85°C (Fig. 7B).

Based on the experimental limits of detection of recA protein by Western blotting (0.1 ng or 2.5 fmol, Fig. 7A lane 5), and the recovery of deproteinized joint molecules (30 fmol, Fig. 7B lane 3), it is estimated that greater than 90% of the joint molecules were free of recA protein (Fig. 7A lane 6). No detectable recA polypeptides were observed by silver staining. In the blot, no recA was observed migrating at the position of recA polypeptides or single-strand or double-strand DNA.

It was also observed that even if active recombinases were present, they did not interfere with branch migration and subsequent dissociation of the DNA molecule. The $^{32}$P-labeled partial duplex structures shown in Fig. 4A were incubated at 37°C with a second oligonucleotide containing a 10 base annealing site in the presence of recA and SSB proteins or HeLa recombinase fraction under joint molecule assay conditions. Branch migrations structures were formed followed by displacement of the $^{32}$P-labeled oligonucleotide within 10 minutes.

Experimental details relating to Examples 6-12.

RecA protein:

Purified E. coli recA protein was provided by Dr. Stephen C. Kowalczykowski, Northwestern University Medical School.
DNA substrates:

pBR322 and pUC18 plasmid DNAs and M13mp18 replicative form DNA were from Pharmacia. Oligonucleotides were synthesized and purified by passage over a Mono Q column (Pharmacia) as described previously (Hsieh and Camerini-Otero, J. Biol. Chem. 264:5089 (1989)). Oligonucleotides were 5'-end labeled with $^{32}$P-gamma-ATP (New England Nuclear) and T4 polynucleotide kinase (Pharmacia) as described previously (Hsieh and Camerini-otero, J. Biol. Chem. 264:5089 (1989)).

Oligonucleotides completely homologous to the plus strand of pBR322 (Figures 11 and 14) spanned pBR322 positions 15-29, 10-29, and 4359-29 for the 15, 20 and 33 base oligonucleotides, respectively (Sutcliffe, *Cold Spring Harbor Symp. Quant. Biol.* 49, 561 (1978)). The 20L series of oligonucleotides (Figure 15) had varying amounts of homology to the plus strand of pBR322 at the 3' end and spanned positions 10-29, 20-29, 22-29, 24-29, and 26-29 for 20, 10, 8, 6, and 4 bases, respectively. The 20R series (Figure 15) had homology to the plus strand of pBR322 at the 5' end and spanned positions 20-39, 20-29, 20-27, 20-25, and 20-23 for 20, 10, 8, 6 and 4 bases, respectively. Oligonucleotides homologous to the plus strand of pUC18 (Figure 10) spanned positions 230-285, 230-267, 230-255, and 230-249 for the 56, 38, 26 and 20 base oligonucleotides, respectively (Norrander et al, *Gene* 26, 101 (1983)). The 33-base oligonucleotide homologous to positions 4359-29 of pBR322 was not paired to the polylinker region of pUC18. The 20 base oligonucleotide used in the experiment shown in Figure 12 was homologous to positions 248-267 of the negative strand of pUC18. The oligonucleotides shown in Figure 13 contained 56 bases homologous to the plus strand of pUC18 corresponding to positions 230-285. The 30 base oligonucleotide homologous to the plus strand of M13mp18 (Figure 16) spanned positions 6831-6860 (Yannisch-Perron et al, *Gene* 33, 103 (1985)). DNA concentrations are expressed as moles of nucleotide or by weight.

Synaptic complex formation:

Synaptic complexes were formed by incubating 1.8 µM (15 ng) oligonucleotide, 18 µM duplex DNA (150 ng) and 1.5 µM (1.5 µg) recA protein in a buffer containing 20 mM Tris-HCl, pH 7.5, 0.4 mM dithiothreitol, 12.5 mM $MgCl_2$, 0.3 mM ATP-gamma-S (Fluka) and 1.1 mM ADP (Sigma) in a total volume of 25 µl for 15 min at 37°C. Following synaptic complex formation, 10-20 units of the appropriate restriction endonuclease (New England Biolabs) were added and incubation continued for an additional 5 min. The reaction was quenched by the addition of SDS and EDTA to a final concentration of 1% and 10 mM, respectively. Reactions were electrophoresed on 1% agarose gels in 40 mM Tris-acetate, pH 8.0, 1 mM EDTA and 1 µg/ml ethidium bromide at 0.6 V/cm for 14-16 h at room temperature. Quantitation was determined by comparison of reacted assays with 150 ng of unreacted duplex DNA using densitometer scanning of Polaroid 665 negatives. Recoveries of intact duplex DNA in synaptic complex assays were compared to a standard containing 150 ng unreacted duplex DNA. In some cases, synaptic complex assays were quenched by the addition of 1% SDS and electrophoresed on 1% agarose gels in 89 mM Tris-borate, pH 8.3, 5 mM $MgCl_2$ at 0.6 V/cm for 14-16 h at room temperature.

Deproteinized joint molecules:

Synaptic complexes were formed as described above except that 5'-$^{32}$P-labeled oligonucleotides were used. The reactions were quenched by the addition of SDS and EDTA and electrophoresed as described. The gels were then fixed and exposed on Kodak XAR-2 film. In some cases, joint molecules were deproteinized by proteinase K (Boehringer Mannheim) treatment and phenol:chloroform extraction as described previously prior to electrophoresis (Hsieh et al, *Genes Devel*. 4, 1951 (1990)). Quantitation of joint molecules was determined by a reconstruction experiment in which a $^{32}$P-labeled 56-base oligonucleotide was annealed to known quantities of M13mp19 single-strand DNA at 65°C or in the presence of recA. (No difference was observed in the efficiency of annealing.) Following electrophoresis and autoradiography, the relative intensities of these annealed standards were compared with those of joint molecules.

EXAMPLE 6

Formation of Synaptic Complexes and Joint Molecules

The experimental scheme for the formation of synaptic complexes and stable joint molecules by recA is shown in Figure 9. Formation of synaptic complexes is accomplished by incubating a duplex DNA such as a supercoiled plasmid DNA, a homologous oligonucleotide and recA protein. The oligonucleotide spans a restriction endonuclease recognition site in the duplex DNA. Formation of a synaptic complex involving recA protein, oligonucleotide and the duplex DNA renders the duplex resistant to cleavage by the restriction endonuclease. The restriction endonuclease footprint corresponding to a synaptic complex can be visualized on ethidium bromide-stained agarose gels as supercoiled plasmid DNA remaining after incubation of complexes with the appropriate restriction endonuclease. RecA protein can be dissociated from these synaptic complexes by adding EDTA and SDS detergent, and deproteinized joint molecules result in which the oligonucleotide (5' end-labeled with $^{32}$P) is stably paired with the duplex. The presence of joint molecules can be determined by assaying for the appearance of $^{32}$P label migrating on agarose gels at the position of duplex DNA.

The formation of joint molecules by recA in the presence of ATP and an ATP regenerating system was examined. It had previously been observed that, under these reaction conditions, stable, deproteinized joint molecules were formed by recA between a linear duplex and a single-strand circular DNA sharing less than 60 bp of homology (Hsieh et al, *Genes Devel*. 4, 1951 (1990)). Stable, deproteinized joint molecules were formed by recA between pUC18 supercoiled DNA and a homologous 56-base oligonucleotide. In the presence of ATP hydrolysis, deproteinized joint molecules were recovered after 1 min but were very unstable; after 3 min, half of the joint molecules had dissociated, and after a 15 min incubation, no deproteinized joint molecules were recovered. Once the initial round of pairing and dissociation had occurred, it appeared that the duplex was unable to participate in additional rounds of pairing. Due to the transient nature of this pairing, footprinting of synaptic complexes was not possible in the presence of ATP. It was observed that replacement of ATP with a nonhydrolyzable analogue of ATP, ATP-gamma-S, and ADP allowed freezing of the pairing reaction and accumulation of intermediates.

The formation of synaptic complexes and stable joint molecules by recA protein in the presence of 0.3 mM ATP-gamma-S and 1.1 mM ADP is shown in Figure 10. $^{32}$P-labeled oligonucleotides homologous to pUC18 plasmid polylinker sequences were incubated with supercoiled pUC18 plasmid DNA in the presence of recA protein followed by the addition of Sac I restriction endonuclease. As depicted in Figure 10A, all the oligonucleotides spanned a unique Sac I restriction endonuclease recognition site in the pUC18 plasmid.

Synaptic complexes were formed with 20 bases of homology shared between the oligonucleotide and the duplex DNA (see Figure 10B, lane 9). Quantitation of the amount of supercoiled DNA protected from digestion by Sac I indicated that 70-75% of the duplex DNA was present as synaptic complexes when the oligonucleotide contained 56 or 38 bases of homology (lanes 3 and 5). Fifty-five percent and 20% of the duplex were converted to synaptic complexes with 26 and 20 bases, respectively, of homology. Formation of synaptic complexes required the presence of both recA protein and the homologous oligonucleotide. A control in lane 1 indicates that when the duplex was incubated with oligonucleotide and recA, but without restriction enzyme, the supercoiled DNA remained intact. As shown in lane 10, the footprint of the synaptic complex did not extend appreciably beyond the region of the duplex that is colinear with the oligonucleotide since synaptic complexes do not afford protection from cleavage by Hind III which cleaves at a site located 14 bp from the region spanned by the 38-base oligonucleotide. In addition, in this assay, a nonhomologous oligonucleotide does not result in the formation of a synaptic complex by recA.

RecA can form joint molecules that are stable when deproteinized between a homologous $^{32}$P-labeled oligonucleotide and pUC18 plasmid DNA (Figure 10C). As few as 26 bases of homology shared between the oligonucleotide and the duplex DNA is sufficient for the formation of joint molecules in this assay whereas twenty bases of homology is not sufficient. Quantitation of the recovery of stable joint molecules indicates that approximately 20-50% of the pUC18 duplex was paired with a homologous oligonucleotide 56 bases long when the complexes were electrophoresed in TAE buffer (see description of Figure 10). As was observed for synaptic complexes, the efficiency of joint molecule formation by recA increases as a function of the length of homology available for pairing. These deproteinized joint molecules dissociate when the superhelical strain is relieved upon linearization at a restriction endonuclease site located outside the region of pairing (Figure 10C, lane 10).

The joint molecules formed between a duplex DNA and an oligonucleotide are not stabilized by residual recA protein. When synaptic complexes were deproteinized by treatment with SDS, proteinase K and phenol/chloroform extraction, the number of stable joint molecules recovered was unchanged.

The assay conditions used for the formation of synaptic complexes were those that proved optimal for joint molecule formation with 56 bp of homology. The formation of joint molecules exhibited a sharp optimum for an oligonucleotide concentration of 1.2 $\mu$M, with 0.9 $\mu$M oligonucleotide yielding no joint molecules; increasing the oligonucleotide concentration to 1.8 $\mu$M resulted in no further increase in the yield of joint molecules. The amount of recA used in these assays (1.5 $\mu$M) is saturating with respect to the single-strand oligonucleotide concentration. The ready detection of synaptic complexes and joint molecules was dependent on the presence of both ATP-gamma-S and ADP in a 1:3 molar ratio. Alteration of the ratio of ATP-gamma-S to ADP or the concentration of these two cofactors reduced the efficiency of both synaptic complex and joint molecule formation.

It is well established that divalent cations are essential for recA activity in vitro. A study was undertaken to determined whether $Mg^{2+}$ is required to stabilize joint molecules. Synaptic complexes were formed in the presence of recA as described in Figure 10. The reactions were deproteinized by the addition of SDS alone and the reaction products analyzed by electrophoresis on agarose gels containing 5mM $MgCl_2$. Although the recovery of joint molecules in the presence of $Mg^{2+}$ was 2-4 fold higher than when $Mg^{2+}$ was omitted from the electrophoresis step, no qualitative differences were observed, i.e., stable joint molecules were formed with oligonucleotides containing 26 bases but not 20 bases of homology.

The data presented in Figure 10 indicate that the formation of synaptic complexes in the presence of ATP-gamma-S is an intermediate step in the pathway leading to the formation of stable, deproteinized joint molecules. The formation of deproteinized joint molecules and synaptic complexes containing recA exhibit a dependence on the length of homology available for pairing. Also, the formation of both synaptic complexes and joint molecules occurs with relatively high efficiency for 56 bases of homology; that is, upon deproteinization of these synaptic complexes, most of the duplex DNA is still paired with the oligonucleotide in stable joint molecules in the presence of $Mg^{2+}$.

EXAMPLE 7

Synaptic Complexes Containing Linear Duplex DNA

Superhelical strain is not essential for the formation of synaptic complexes involving very short regions of homology. The formation of synaptic complexes involves recA, a linear duplex and a homologous oligonucleotide 33 bases long (Figure 11A). In Figure 11B, it is readily seen that recA formed synaptic complexes between these two substrates resulting in protection of the linear duplex from cleavage by Cla I (lane 4). In the absence of either recA or oligonucleotide (lanes 2 and 6, respectively) or in the presence of a nonhomologous oligonucleotide (lane 5), synaptic complexes were not formed.

EXAMPLE 8

Footprinting Synaptic Complexes

The extent of protection from restriction endonuclease cleavage conferred by a recA synaptic complex was mapped (Figure 12). A 20-base oligonucleotide homologous to a region in the polylinker sequence of pUC18 plasmid DNA was incubated in the presence of supercoiled pUC18 and recA to allow the formation of synaptic complexes. Protection was seen at sites for Bam HI, Kpn I, Pst I, Sac I and Sph I restriction endonucleases. However, cleavage by Eco RI and Hind III was unimpaired by the presence of the synaptic complex. Accordingly, the footprint of the synaptic complex apparently extends approximately 13-14 bases beyond the 5' and 3' ends of the paired oligonucleotide and is symmetrical.

EXAMPLE 9

Directionality of Synaptic Complex and Joint Molecule Formation

The apparent directionality of joint molecule formation is influenced by the choice of DNA substrates, the length of shared homology and the relative stabilities of joint molecules formed with opposite polarities (Konforti et al, *J. Biol. Chem*. 265, 6916 (1990); Rao et al, *Proc. Natl. Acad. Sci*. 88, 2984 (1991)). Therefore, the directionality of both synaptic complex formation and joint molecule formation involving a supercoiled duplex and an oligonucleotide was examined. The formation of synaptic complexes involving 56 bp of homology does not exhibit directionality. Synaptic complexes were formed by recA regardless of the positioning of the homologous sequence with respect to the ends of the oligonucleotide. In the experiment in Figure 13, a 56-base oligonucleotide homologous to the polylinker region of pUC18 was used or one of several other oligonucleotides having the same 56-base sequence plus 20 bases of nonhomologous sequence at the 5' end, at the 3' end or at both the 5' and 3' ends of the oligonucleotide (Figure 13A). In all cases, synaptic complexes were formed with about equal efficiencies (Figure 13B, lanes 5, 7, 9, 11). In contrast, formation of stable joint molecules by recA exhibited polarity showing a strong preference for homology at the 5' end of the oligonucleotide; the number of joint molecules formed with the 76R oligonucleotide was half as many as with the 56-base oligonucleotide (Figure 13C, lanes 2 and 6) whereas the 76L oligonucleotide (lane 4) or 96 oligonucleotide (lane 8) yielded tenfold fewer joint molecules.

EXAMPLE 10

Minimum Structure Required for the Homology Search

Three oligonucleotides, 33, 15 or 13 bases long and homologous to a pBR322 sequence (Figure 14A) were incubated with pBR322 supercoiled plasmid DNA in the presence of recA. Potential cleavage sites for the 15 base oligomer are shown in Figure 14B and the footprint is shown in Figure 14C. The 15 base oligomer was of sufficient length to form synaptic complexes as evidenced by resistance to cleavage by Hind III and Cla I endonucleases (lanes 4 and 7). In this assay, use of a 33-base oligomer resulted in the formation of synaptic complexes, but a 13-base oligomer did not. Control experiments indicate that the formation of synaptic complexes required the presence of both oligonucleotide and recA. The footprint of the synaptic complex did not extend appreciably beyond the region of pairing (see Figure 14C, lane 10). This experiment also demonstrates that formation of synaptic complexes by recA was not restricted to any particular sequence since recA paired homologous DNAs containing either a pUC18 (Figure 10) or a pBR322 sequence (Figure 11).

EXAMPLE 11

Nucleation of Pairing Involves One-Half of a Helical Turn of the Nucleoprotein Filament

To determine the minimum homology recognized by recA in a synaptic complex, a series of oligonucleotides 20 bases long was used that contained varying amounts of homology at the 5' or 3' end to a region of pBR322 flanking a Cla I site (see Table I). This experiment not only establishes the minimum homology recognized by recA in this assay, but also definitively establishes whether the initiation of pairing by recA exhibits directionality. The results shown in Figure 15 indicate that recA can pair as few as 8 bases of homology at either the 5' or 3' end albeit at low efficiency (10% and 12%, respectively). These results establish that the thresholds for nucleoprotein filament formation and homologous pairing are different and that either the 5' or 3' end of a single-strand DNA can nucleate pairing. Fifteen bases are required to form the structure that can carry out the homology search, but only one-half of the bases in this structure need be recognized and paired by recA.

## Table 1. SEQUENCES OF OLIGONUCLEOTIDES CONTAINING DECREASING AMOUNTS OF HOMOLOGY TO pBR322

### 20L Series

| Sequence | Homology (bases) |
|---|---|
| 5' TTGACAGCTTATCATCGATA 3' | 20 |
| GAATATATGCATCATCGATA | 10 |
| GAATATATGCCACATCGATA | 8 |
| GAATATATGCCATGTCGATA | 6 |
| GAATATATGCCATGGAGATA | 4 |
| GAATATATGCCATGGATCGT | 0 |

### 20R Series

| Sequence | Homology (bases) |
|---|---|
| ATCATCGATAAGCTTTAATG | 20 |
| ATCATCGATAGAATATATGC | 10 |
| ATCATCGAGCGAATATATGC | 8 |
| ATCATCTCGCGAATATATGC | 6 |
| ATCAGATCGCGAATATATGC | 4 |
| CGACGATCGCGAATATATGC | 0 |

Sequences in bold correspond to the bases homologous to pBR322.

Underlined sequences correspond to the position of the Cla I restriction site on the duplex.

EXAMPLE 12

Targeting of an Oligonucleotide by recA

A study was undertaken to determine to what extent recA can discriminate among several similar but distinct target sequences that reside within a single duplex molecule. Supercoiled M13mp18 replicative form DNA which has three Nde I recognition sites was incubated in the presence of recA with a 30-base oligonucleotide containing the Nde I recognition sequence as well as adjacent sequence from one of the three Nde I sites in M13mp18 (site I, see Figure 16A). The formation of a synaptic complex exclusively at site I was monitored by the appearance of a 6170 bp M13mp18 fragment following digestion of synaptic complexes with Nde I endonuclease. Such a fragment can only come about by cleavage at both sites II and III without cleavage at site I.

RecA was able to target pairing exclusively to site I in the majority of the DNA molecules (Figure 16B, lane 5). Such targeting required the presence of both oligonucleotide and recA (lanes 3 and 4). The presence of a 1080 bp fragment in all samples incubated with Nde I is a control for the extent of Nde I cleavage at both unprotected sites II and III. Quantitation of the amounts of each species in lane 5 indicates that the level of discrimination of recA for pairing at the target site I over sites II and III is about 7-8 fold under these conditions.

Experimental details relating to Examples 13-15.

Oligonucleotides:

Oligonucleotides were purified on an FPLC Mono Q column (Pharmacia) using a NaCl gradient from 100 mM to 1 M in 20 mM NaOH. Aliquots of peak fractions were labeled with $^{32}$P and run on polyacrylamide gels. The purest fractions were pooled, ethanol precipitated, and dissolved in water. Concentrations were determined by assuming that 1 OD unit at 260 nM is 33 $\mu$g.

RecA:

RecA protein was purified using a strain and a detailed protocol generously provided by Stephen Kowalczykowski of the Northwestern University Medical School in Chicago. The strain used was JC12772 (Uhlin et al, *J. Bacteriol.* 148, 386 (1981)). The purification was based on the spermidine precipitation method (J. Griffith et al, *Biochemistry* 24, 158 (1985)), and employed a single-stranded DNA agarose column with ATP elution (Cox et al *J. Biol. Chem.* 256, 4676 (1981)) and a Mono Q column to greatly reduce trace nuclease contamination. Removal of such contamination is important in order to avoid undesirable non-specific nicking of the DNA. The concentration of recA protein was measured using the extinction coefficient of $^{1\%}E_{280}$ = 5.9 (Craig et al, *J. Biol. Chem.* 256, 8039 (1981)).

EXAMPLE 13

Sequence Specific Cleavage of Lambda DNA

A demonstration of the sequence-specific cleavage of lambda DNA at a single site is shown in Figure 18. Lambda DNA is 48.5 kb in length, and contains 5 Eco RI sites (Daniels et al, in *Lambda II*, Hendrix et al, Eds. (Cold Spring Harbor, N.Y. 1983), pp. 519-678). The site located at nucleotide position 31,747 was selected for cleavage in order to cut lambda into two fragments of 31.7 and 16.8 kb. An oligonucleotide 30 bases long and homologous to this position was synthesized, and Figure 18B shows the results of the cleavage using this oligonucleotide. Lane 1 shows uncut lambda DNA, and lane 2 shows a complete cleavage experiment. Densitometry of lane 2 showed that 79% of the DNA was cleaved into the desired two fragments, and 19% of the DNA was uncut. A total of 2% of the DNA was cut at one of the four other Eco RI sites present in lambda DNA, caused by either nonspecific methylation protection by the recA protein and oligonucleotide complex, or incomplete methylation. Controls in lanes 3, 4, and 5 show the result of omitting recA protein, oligonucleotide, or methylase, respectively. Notably, in lane 3, omitting the recA protein resulted in incomplete methylation, possibly due to inhibition of the methylase by free oligonucleotide not coated with recA protein. Lane 4 DNA also showed slightly incomplete methylation, possibly because some nonspecific protection occurred from free recA protein binding to the duplex lambda DNA. This effect was seen more dramatically in Figures 19 and 20 where an oligonucleotide titration was done.

EXAMPLE 14

Sequence Specific Cleavage of E. coli DNA

Experimental details:

Wild type E. coli strain W3110 was obtained from the American Type Culture Collection and was grown overnight in Luria-Bertani medium to an optical density (OD) at 600 nm of 5. 5 ml of cells were pelleted (30 mg wet weight), washed once with 10 mM Tris-HCl (pH 7.2), 20 mM NaCl, and 100 mM EDTA, and resuspended in 1 ml of this buffer. The suspension was brought to 65°C, and added to 1 ml of 1.6% low melting point agarose (InCert agarose, FMC Bioproducts) and 4 ml of paraffin oil at 65°C. Microbeads 25 to 100 μm in diameter were formed by vortexing the suspension as described (M. McClelland, *Methods Enzymol*. 155, 22 (1987)). Beads were digested with lysozyme and proteinase K using the ImBed kit (New England Biolabs) following the manufacturer's directions. Other lysozyme and proteinase K preparations gave equally good results. Beads were stored at 4°C. and were incubated at 50 mM EDTA for 30 minutes and equilibrated in 25 mM Tris-acetate (pH 7.5), 4 mM Mg-acetate, 0.4 mM dithiothreitol, and 0.5 mM spermidine immediately prior to use.

Results:

Application of the cleavage reaction to E. coli DNA is shown in Figure 19. In this case, a pair of oligonucleotides was added to obtain a fragment by cleavage at two sites. A large fragment was generated to test the power of the method. As shown in Figure 19A, one oligonucleotide was homologous to the uvrB gene, and the other to the topA gene. The oligonucleotides spanned Eco RI sites in each of these genes. The two genes are located 520 kb apart on the chromosome (Rudd et al, *Nucl. Acids Res*. 18, 313 (1990)), and at least 67 Eco RI sites are between these two target sequences (Kohara et al, *Cell* 50, 495 (1987)). Figure 19B shows the expected 520 kb band. A fairly sharp optimum was observed for oligonucleotide concentration of 5 nucleotide residues per recA protein monomer (lane 2). This was more clearly seen in the Soutbern blot in Figure 19C. Densitometry of the blot gave a yield of the fragment of 40%. As in the cleavage of lambda DNA, there was some non-specific protection from methylation by recA protein at lower oligonucleotide concentrations, and the 520 kb fragment was cleaved into smaller fragments. At higher oligonucleotide concentrations, the 520 kb fragment was also cleaved into smaller fragments, as would be expected from the result with lambda DNA in lane 3 of Figure 18B. An identical pattern with an optimum of 5 nucleotide residues per recA protein monomer was seen when the length of the oligonucleotides was increased from 30 to 60 bases, only in this case the yield of the 20 kb fragment increased to 60%. The 40 and 60% yields for the different pairs of oligonucleotides correspond to minimum single-side cutting efficiencies of 63 and 77%, respectively. This is close to the cutting efficiency on lambda DNA of 79%.

In certain applications, sequence information on both sides of an Eco RI site might be difficult to obtain. The fragment yield was therefore measured when the Eco RI recognition sequence, GAATTC, was at the 5' or the 3' end of a pair of oligonucleotides, instead of in the middle as in the previous study. When the recognition sequence was at the 5' end of the oligonucleotides (30 bases in length), the yield dropped two- to fourfold. When the sequence was at the 3' end, the yield dropped an additional twofold.

EXAMPLE 15

Sequence Specific Cleavage of Human DNA

Experimental Details:

Beads containing HeLa cell DNA were prepared by washing $1 \times 10^8$ cells (150mg wet weight) twice with phosphate buffered isotonic saline and processed as in Example 9 for the E. coli beads, except that the lysozyme digestion step was omitted.

Results:

The cleavage reaction was performed on human DNA with similar success. As the cystic fibrosis (CF) locus has been extensively mapped and sequenced (Rommons et al, *Science* 245, 1059 (1989); Riordan et al, *Science* 245, 1066 (1989), Zielenski et al, *Genomics* 10, 214 (1991)), it was used as a locus to test the method. Figure 20A is a simple schematic of the CF locus. An Eco RI site is present in intron 1, and is 180 kb away from another Eco RI site in exon 19. At least 41 other Eco RI sites are found on this 180 kb stretch of genomic DNA (Rommons et al, *Science* 245, 1059 (1989)). A gel stained with ethidium bromide is shown in Figure 20B, and shows how the production of smaller fragments occurred when the oligonucleotide concentration was lowered. This pattern was very reproducible and could be used as a guide to find the optimal concentration of oligonucleotide without doing Southern blotting. The Southern blot of the gel is shown in Figure 20C. The greatest yield of the fragment was found in lane 3 (86%). A smaller yield (32%) was found in lane 2, but the background cleavage in lane 2 was much lower than in lane 3. Thus, DNA from the 180 kb region of lane 2 probably was the most enriched in DNA from the CF locus. A control shown in lane S is the 270 kb fragment produced by digestion with Sfi I (Rommons et al, *Science* 245, 1059 (1989)). A predicted 48 kb fragment could also be produced by specific cleavage at exons 13 and 19.

It was also noted in Figure 20C that in lanes 3-6, the 180 kb fragment was further broken down to smaller fragments. These fragments were probably generated by one specific cleavage at intron 1 or exon 19, and one non-specific cleavage of the fragment internally. As the probe used is 50 kb from the exon 19 site, no fragments under 50 kb in length hybridized, although presumably they were present.

The entire contents of all references cited above are incorporated herein by reference.

Certain aspects of the present invention have been described in some detail for purposes of clarity and understanding. One skilled in the art will appreciate, however, that various changes can be made in form and detail without departing from the true scope of the invention.

**Claims**

1. A method of effecting cleavage of a double-stranded DNA molecule at a specific site comprising the steps of :

    i) contacting a homologous recombination protein with a double-stranded DNA molecule and with a single-stranded DNA molecule sufficiently complementary to a portion of said double-stranded DNA molecule to hybridize therewith, wherein said contacting is effected under conditions such that said single-stranded DNA molecule hybridizes to said double-stranded molecule so that a DNA triplex is formed,
    wherein, where said hybridization occurs, each strand of said DNA triplex is hybridized to at least one other other strand of said DNA triplex,
    wherein one end of said single-stranded DNA molecule has attached thereto a cleavage moiety, which end is adjacent to said specific cleavage site ; and
    ii) exposing said cleavage moiety to conditions such that said cleavage moiety effects cleavage at said specific cleavage site.

2. The method according to claim 1 wherein said recombination protein is a bacterial or bacteriophage protein.

3. The method according to claim 2 wherein said protein is E. coli recA.

4. The method according to claim 1 wherein said recombination protein is a eucaryotic recombinase.

5. The method according to claim 4 wherein said recombinase protein is a human recombinase protein.

6. The method according to claim 1 wherein said cleavage moiety is a chelating agent.

7. The method according to claim 1 wherein said cleavage moiety is a light-activated dye.

8. A method of effecting cleavage of a double-stranded DNA molecule containing at least two restriction endonuclease recognition sites at a first of said sites by a restriction enzyme specific for said sites comprising :

    i) contacting a recombination protein with the double-stranded DNA molecule and with a single-stranded DNA molecule, which single-stranded DNA molecule is sufficiently complementary to a portion of one strand of said double-stranded DNA molecule that includes the first of said restriction endonuclease recognition sites to hybridize therewith,
    wherein said contacting is effected under conditions such that said single-stranded DNA molecule hybridizes to said double-stranded molecule at said first of said restriction endonuclease recognition sites so that a three-stranded DNA molecule is formed ;
    ii) modifying the at least one other of said sites so as to render it protected from said restriction enzyme ;
    iii) dissociating said single-stranded DNA molecule from said double-stranded molecule ; and
    iv) cleaving said double-stranded molecule resulting from step (iii) at said first of said restriction sites.

9. The method according to claim 8 wherein said contacting of step (i) is effected in the presence of ATP-gamma-S and ADP.

10. The method according to claim 9 wherein ATP-gamma-S and ADP are present during said contacting of step (i) in a molar ratio of about 1:3.

11. The method according to claim 9 wherein ADP is present during said contacting of step (i) at a concentration of about 1.1mM.

12. The method according to claim 9 wherein ATP-gamma-S is present during said contacting of step (i) at a concentration of about 0.3mM.

13. The method according to claim 8 wherein said contacting of step (i) is effected in the presence of $Mg^{2+}$ and spermidine.

14. The method according to claim 13 wherein the concentration of magnesium is 4mM.

15. The method according to claim 9 wherein said contacting of step (i) is effected in the presence of $Mg^{2+}$.

**Patentansprüche**

1. Verfahren zur Durchführung des Spaltens eines doppelsträngigen DNA-Moleküls an einer spezifischen Stelle, die Schritte umfassend:

i) Inkontaktbringen eines homologen Rekombinations-Proteins mit einem doppelsträngigen DNA-Molekül und einem einzelsträngigen DNA-Molekül, das ausreichend komplementär zu einem Teil des doppelsträngigen DNA-Moleküls ist, um damit zu hybridisieren, wobei das Inkontaktbringen unter Bedingungen durchgeführt wird, bei denen das einzelsträngige DNA-Molekül mit dem doppelsträngigen DNA-Molekül hybridisiert, so daß eine DNA-Triplex gebildet wird,
wobei, wenn die Hybridisierung stattfindet, jeder Strang der DNA-Triplex mit mindestens einem anderen Strang der DNA-Triplex hybridisiert ist,
wobei an ein Ende des einzelsträngigen DNA-Moleküls eine Spaltungseinheit angebracht ist, wobei das Ende zu der spezifischen Schnittstelle benachbart ist; und
ii) Unterwerfen der Spaltungseinheit unter Bedingungen, so daß die Spaltungseinheit das Spalten an der spezifischen Schnittstelle bewirkt.

2. Verfahren nach Anspruch 1, wobei das rekombinante Protein ein bakterielles oder Bakteriophagen-Protein ist.

3. Verfahren nach Anspruch 2, wobei das Protein E. coli recA ist.

4. Verfahren nach Anspruch 1, wobei das Rekombinations-Protein eine eukaryontische Rekombinase ist.

5. Verfahren nach Anspruch 4, wobei das Rekombinase-Protein ein menschliches Rekombinase-Protein ist.

6. Verfahren nach Anspruch 1, wobei die Spaltungseinheit ein Chelatbildner ist.

7. Verfahren nach Anspruch 1, wobei die Spaltungseinheit ein Licht-aktivierter Farbstoff ist.

8. Verfahren zum Durchführen des Spaltens eines doppelsträngigen DNA-Moleküls, das mindestens zwei Restriktionsendonuclease-Erkennungsstellen enthält, an einer ersten der Stellen durch ein Restriktionsenzym, das spezifisch für diese Stellen ist, umfassend:

i) Inkontaktbringen eines Rekombinations-Proteins mit dem doppelsträngigen DNA-Molekül und einem einzelsträngigen DNA-Molekül, wobei das einzelsträngige DNA-Molekül ausreichend komplementär zu einem Teil eines Strangs des doppelsträngigen DNA-Moleküls ist, das die erste der Restriktionsendonuclease-Erkennungsstellen einschließt, um damit zu hybridisieren,
wobei das Inkontaktbringen unter Bedingungen durchgeführt wird, so daß das einzelsträngige DNA-Molekül mit dem doppelsträngigen DNA-Molekül an der ersten der Restriktionsendonuclease-Erkennungsstellen hybridisiert, so daß ein dreisträngiges DNA-Molekül gebildet wird;
ii) Modifizieren der mindestens einen anderen der Stellen, um sie so zu verändern, daß sie vor dem Restriktionsenzym geschützt ist;
iii) Dissoziation des einzelsträngigen DNA-Moleküls von dem doppelsträngigen DNA-Molekül; und
iv) Spalten des aus Schritt (iii) folgenden doppelsträngigen Moleküls an der ersten der Restriktionsstellen.

**9.** Verfahren nach Anspruch 8, wobei das Inkontaktbringen des Schritts (i) in Gegenwart von ATP-gamma-S und ADP durchgeführt wird.

**10.** Verfahren nach Anspruch 9, wobei ATP-gamma-S und ADP während des Inkontaktbringens von Schritt (i) in einem molaren Verhältnis von etwa 1:3 vorliegen.

**11.** Verfahren nach Anspruch 9, wobei ADP während des Inkontaktbringens von Schritt (i) mit einer Konzentration von etwa 1,1 mM vorliegt.

**12.** Verfahren nach Anspruch 9, wobei ATP-gamma-S während des Inkontaktbringens von Schritt (i) mit einer Konzentration von etwa 0,3 mM vorliegt.

**13.** Verfahren nach Anspruch 8, wobei das Inkontaktbringen von Schritt (i) in Gegenwart von $Mg^{2+}$ und Spermidin durchgeführt wird.

**14.** Verfahren nach Anspruch 13, wobei die Magnesiumkonzentration 4 mM beträgt.

**15.** Verfahren nach Anspruch 9, wobei das Inkontaktbringen von Schritt (i) in Gegenwart von $Mg^{2+}$ durchgeführt wird.

**Revendications**

**1.** Procédé de coupure d'une molécule d'ADN double brin à un site spécifique, comprenant les étapes selon lesquelles:

i) on met en contact une protéine de recombinaison homologue avec une molécule d'ADN double brin et avec une molécule d'ADN simple brin suffisamment complémentaire d'une portion de ladite molécule d'ADN double brin pour s'y hybrider, en effectuant ladite mise en contact dans des conditions telles que ladite molécule d'ADN simple brin s'hybride à ladite molécule double brin pour former une triple hélice d'ADN, chaque brin de ladite triple hélice d'ADN étant hybridé à au moins un autre brin de ladite triple hélice d'ADN lorsque ladite hybridation se produit; un fragment de coupure étant lié à une extrémité de ladite molécule d'ADN simple brin, cette extrémité étant adjacente audit site de coupure spécifique; et ii) on expose ledit fragment de coupure à des conditions telles que ledit fragment de coupure effectue une coupure audit site de coupure spécifique.

**2.** Procédé selon la revendication 1, dans lequel ladite protéine de recombinaison est une protéine bactérienne ou une protéine de bactériophage.

**3.** Procédé selon la revendication 2, dans lequel ladite protéine est recA d'*E. coli*.

**4.** Procédé selon la revendication 1, dans lequel ladite protéine de recombinaison est une recombinase eucaryote.

**5.** Procédé selon la revendication 4, dans lequel ladite recombinase est une recombinase humaine.

**6.** Procédé selon la revendication 1, dans lequel ledit fragment de coupure est un agent chélatant.

**7.** Procédé selon la revendication 1, dans lequel ledit fragment de coupure est un colorant activé par la lumière.

**8.** Procédé de coupure d'une molécule d'ADN double brin contenant au moins deux sites de reconnaissance d'une endonucléase de restriction à un premier de ces sites par une enzyme de restriction spécifique de ces sites, comprenant:

i) la mise en contact d'une protéine de recombinaison avec la molécule d'ADN double brin et avec une molécule d'ADN simple brin, cette molécule d'ADN simple brin étant suffisamment complémentaire d'une portion d'un brin de ladite molécule d'ADN double brin comprenant le premier desdits sites de reconnaissance d'endonucléase de restriction pour s'y hybrider, cette mise en contact étant effectuée dans des conditions telles que ladite molécule d'ADN simple brin s'hybride à ladite molécule double brin au niveau du premier desdits sites de reconnaissance d'endonucléases de restriction, de façon à former une molécule d'ADN triple brin;

ii) la modification de l'autre ou des autres desdits sites de manière à les protéger de ladite enzyme de restriction;

iii) la dissociation de ladite molécule d'ADN simple brin de ladite molécule double brin; et

iv) la coupure de ladite molécule double brin obtenue dans l'étape (iii) au niveau du premier desdits sites de restriction.

9. Procédé selon la revendication 8, dans lequel ladite mise en contact de l'étape (i) s'effectue en présence d'ATP-gamma-S et d'ADP.

10. Procédé selon la revendication 9, dans lequel l'ATP-gamma-S et l'ADP sont présents pendant ladite mise en contact de l'étape (i) dans un rapport molaire d'environ 1:3.

11. Procédé selon la revendication 9, dans lequel l'ADP est présent pendant ladite mise en contact de l'étape (i) à une concentration d'environ 1,1 mM.

12. Procédé selon la revendication 9 dans lequel l'ATP-gamma-S est présent pendant ladite mise en contact de l'étape (i) à une concentration d'environ 0,3 mM.

13. Procédé selon la revendication 8, dans lequel ladite mise en contact de l'étape (i) steffectue en présence de $Mg^{2+}$ et de spermidine.

14. Procédé selon la revendication 13, dans lequel la concentration du magnésium est de 4 mM.

15. Procédé selon la revendication 9, dans lequel la mise en contact de l'étape (i) s'effectue en présence de $Mg^{2+}$.

5'
3'

**FIG. 1A**

5'
3'

**FIG. 1B**

FIG. 2A

FIG. 2B

FIG. 2C

FIG. 2D

FIG. 3A

origin →

joint molecule →

$^{32}P$ oligo →

FIG. 3B

**Duplex**

# FIG. 4A

**Branch Migration**

# FIG. 4B

**Joint Molecule**

RECOM BINASE SDS
10' 37° EDTA

# FIG. 4C

EP 0 556 330 B1

0° 37° 45° 55° 65° 75° 85°

origin→

M13mp19→

$^{32}$P oligo→

**FIG. 4D**

0° 37° 45° 55° 65° 75° 85°

origin→

M13mp19→

$^{32}$P oligo→

**FIG. 4E**

0°   37°   45°   55°   65°   75°   85°

joint
molecule→

ds→

ss→

**FIG. 4F**

FIG. 5

FIG. 6A

pGEM 4

M13mp18

5'
3'

+

–

56 bp

BglI

Hind III

0° 45° 55° 65° 75° 85°

ORIGIN→

JOINT
MOLECULE→

ds→

FIG. 6B

FIG. 6C

FRACTION REMAINING

●RecA
○Hela
△BRANCH MIGRATION

T (°C)

FIG. 7A

FIG. 7B

FIG. 8A

FIG. 8B-1

FIG. 8B-2

FIG. 8B-3

FIG. 8B-4

**FIG. 9**

EP 0 556 330 B1

HindIII

SacI

## FIG. 10A

5' ...AAGCTTGCATGCCTGCAGGTCGACTCTAGAGGATCCCCGGGTACCGAGCTCGAATTC...
3' ...TTCGAACGTACGGACGTCCAGCTGAGATCTCCTAGGGGCCCATGGCTCGAGCTTAAG...

HindIII

SacI

3'                                                                                  5'
TCGAACGTACGGACGTCCAGCTGAGATCTCCTAGGGGCCCATGGCTCGAGCTTAAG  56
                                                                                    38
                                                                                    26
                                                                                    20

```
        1   2   3   4   5   6   7   8   9   10
oligo  20  -56-    -38-    -26-    -20-    38
RecA    +   -   +   -   +   -   +   -   +   +
SacI    -   +   +   +   +   +   +   +   +  HindIII
```

## FIG. 10B

I→

sc→

1   2   3   4   5   6   7   8   9   10

## FIG. 10C

sc→

Cla I

33

NruI ——————————————————————— NruI

3414 bp                    949 bp

## FIG. 11A     pBR322

| LANES | 1 | 2 | 3 | 4 | 5 | 6 |
|-------|---|---|---|---|---|---|
| OLIGO | - | H | N | H | N | - |
| REC A | - | - | - | + | + | + |
| Cla I | - | + | + | + | + | + |

4363 bp →
3413 bp →

949 bp →

## FIG. 11B

EP 0 556 330 B1

17

EcoRI       Sac     7     3'   Bam              5'     Pst     Sph Hind    12 14

Kpn **GCCCCTAGGAGATCTCAGCT**

5' . . . G A A T T C G A G C T C G G T A C C **C G G G G G A T C C T C T A G A G T C G A** C C T G C A G G C A T G C A A G C T T . . .

3' . . . C T T A A G C T C G A G C C A T G G **G C C C C T A G G A G A T C T C A G C T** G G A C G T C C G T A C G T T C G A A . . .

EcoRI Sac    Kpn     1       Bam          20 Pst    Sph      Hind

13 11                             8       18

## FIG. 12

```
56                                            5' ─────────────56──────────────→ 3'
76L  5' ACTGCGTAACGGTAGCATGA ───────────────────────────────→ 3'
76R                                           5' ─────────────────────────ACCGATCTGACGTGAGTGAC 3'
96   5' ACTGCGTAACGGTAGCATGA ─────────────────────────────ACCGATCTGACGTGAGTGAC 3'
```

# FIG. 13A

# FIG. 13B

# FIG. 13C

13

15

33

E    C    H

pBR322

## FIG. 14A

5' AGCTTATCATCGATA 3'

## FIG. 14B

5' ...AGCTTATCATCGATA...
3' ...TCGAATAGTAGCTAT...

ClaI  HindIII

| LANES | 1 | 2 | 3 | 4 | 5 | 6 | 7 | 8 | 9 | 10 |
|---|---|---|---|---|---|---|---|---|---|---|
| oligo 15b. | - | + | - | + | + | - | + | + | - | + |
| RecA | - | - | + | + | - | + | + | - | + | + |
| REST. ENZ. | - | HindIII | | | ClaI | | | EcoRI | | |

## FIG. 14C

FIG. 15A

FIG. 15B

44

NdeI

30

N

3043 bp    M13mp18    3127 bp

III    II

N 1080bp N

6170 bp

M13mp18

1080 bp

## FIG. 16A

| Lanes | M | 1 | 2 | 3 | 4 | 5 | 6 |
|-------|---|---|---|---|---|---|---|
| Oligo | – | – | – | + | – | + | – |
| RecA | – | – | – | – | + | + | – |
| NdeI | – | – | + | + | + | + | BamHI |

←6170

←3127
←3043

←1080

## FIG. 16B

DNA with EcoRI sites

Bind recA protein and
oligonucleotide complex

Add methylase

Remove proteins and oligonucleotide
and digest with EcoRI

FIG. 17

↑ EcoRI site
X methylated EcoRI site
-OOO- recA protein with
an oligonucleotide

EP 0 556 330 B1

λ DNA
48.5 kb

31.7          16.8

↑ EcoRI sites
↑ oligo overlap site

FIG. 18A

| Lanes | 1 | 2 | 3 | 4 | 5 | 6 |
|---|---|---|---|---|---|---|
| RecA | − | + | − | + | + | − |
| Oligo | − | + | + | − | + | − |
| Methylase | − | + | + | + | − | − |
| EcoRI | − | + | + | + | + | + |

← 48.5 (kb)
← 31.7
← 21.2
← 16.8
← 7.4

FIG. 18B

E. coli
4,720 kb

uvrB

520 kb

topA

## FIG. 19A

Oligo(ng)    680 460 310 200 140

Lanes   Y  λ  E  1  2  3  4  5

← C

← 520
(kb)

## FIG. 19B

E  1  2  3  4  5

## FIG. 19C

CF GENE

Exon 1  Introns 2-17 Exons 2-18  Exon 19

180 kb

## FIG. 20A

Oligo(ng)  1,340 900 600 400 270 180

Lanes  S λ 1 2 3 4 5 6

## FIG. 20B

S λ 1 2 3 4 5 6

270 →
180 →
(kb)

## FIG. 20C

EP 0 556 330 B1